# EUROPÄISCHE PATENTSCHRIFT

(11) **EP 2 994 201 B1**
(45) Veröffentlichungstag und Bekanntmachung des Hinweises auf die Patenterteilung: **20.12.2017**
(21) Anmeldenummer: 14724375.2
(22) Anmeldetag: 06.05.2014
(51) Int. Cl.: A61Q 17/04, A61K 8/37, A61K 8/49

(54) **MITTEL MIT HOHEN MENGEN AN UV-STABILISATOREN**
COMPOSITIONS WITH HIGH QUANTITIES OF UV STABILIZERS
AGENTS CONTENANT DES QUANTITÉS ÉLEVÉES DE STABILISATEURS UV

(30) Priorität: 10.05.2013 EP 13167285
(43) Veröffentlichungstag der Anmeldung: 16.03.2016
(73) Patentinhaber: BASF SE, 67056 Ludwigshafen am Rhein (DE)
(72) Erfinder: HLOUCHA, Matthias, 50677 Köln (DE); KÜSTERS, Esther, 40699 Erkrath (DE); ACKER, Stephanie, F-68440 Dietwiller (FR); FLÖßER-MüLLER, Heike, B-2930 Brasschaat (BE); SCHORB, Jasmin, 40789 Monheim (DE); SEIDLER, Stefanie, 40597 Düsseldorf (DE); DÖRFLINGER, Tatjana, 79692 Kleines Wiesental (DE)
(74) Vertreter: Upschulte, Manfred Alois
(86) Internationale Anmeldenummer: PCT/EP2014/059187
(87) Internationale Veröffentlichungsnummer: WO 2014/180818

(56) Entgegenhaltungen:
- EP-A1- 2 426 101
- EP-A1- 2 517 689
- EP-A2- 0 813 861
- WO-A1-03/088941
- WO-A2-03/075879
- WO-A2-2012/156109
- DE-A1- 19 737 737
- DE-A1- 19 937 299
- US-B1- 6 306 373

## Beschreibung

Die vorliegende Erfindung betrifft Mittel für die Herstellung von hochkonzentrierten Formulierungen mit hohen Mengen an UV- Filtern, ein Verfahren zur Herstellung von Sonnenschutz-Endformulierungen, basierend auf derartigen Mitteln sowie die Verwendung derartiger Mittel für die Herstellung von kosmetischen Formulierungen mit extrem hohen Sonnenschutzfaktoren (SPF bis 50+).

Aus dermatologischer Sicht sollen Sonnenschutzformulierungen neben dem pflegenden Effekt für die Haut vor allem die negativen Wirkungen der Sonneneinstrahlung mindern bzw. soweit wie möglich verhindern. Dazu enthalten die Sonnenschutzformulierungen UV- Filter, die über Wirksamkeit, Menge und Kombination den UV-Schutz, meist ausgedrückt als SPF-Faktor, bestimmen.

Für den Hersteller solcher Sonnenschutzformulierungen ist es wichtig, dass die UV- Filter in einer lagerstabilen Form vorliegen, die leicht und möglichst bei Raumtemperatur in eigene, definierte Sonnenschutz-Endformulierungen überführt werden können. Durch einfaches Abmischen möchte der Hersteller eine pflegende O/W- Emulsion erhalten, deren SPF-Faktor nach eigenen Wünschen bestimmbar ist.

Aus der Offenlegungsschrift DE 102005011785 sind kosmetische O/W-Emulsionen bekannt, die auch Lichtschutzfilter enthalten können. Diese O/W-Emulsionen zeichnen sich durch eine Emulgatorkombination aus Nichtkohlenhydrat-Polyolpartialestern von Fettsäuren, insbesondere Polyglycerinpartialestern, und Emulgatoren auf Kohlenhydratbasis, insbesondere Glykosiden, aus. Mit Hilfe diesen speziellen Emulgatorkombinationen werden zunächst Emulsionskonzentrate hergestellt, die anschliessend schnell mit Wasser verdünnt werden müssen, da sie nicht lagerstabil sind.

Aufgabe der vorliegenden Erfindung ist es, Mittel mit hohen Mengen an UV-Filtern zur Verfügung zu stellen, die lagerstabil sind und somit auch erst nach Wochen mit Wasser in O/W-Emulsionen überführt werden können. Die Mittel sollen als Konzentrate einsetzbar sein, d.h. hohe Mengen an UV-Filter sollen in hohen Mengen an Ölen gelöst sein, ohne dass es bei längerer Lagerung zur Auskristallisierung dieser UV-Filter kommt. Auch ist gewünscht, dass diese Mittel im Sinne eines Konzentrats möglichst wenig Wasser enthalten. Des Weiteren sollen sich die Mittel leicht und ohne besondere Scherkräfteeinwirkung mit Wasser in O/W-Emulsionen für den Sonnenschutz überführen lassen.

Überraschenderweise konnte die Aufgabe gelöst werden durch hochkonzentrierte Mittel für die Herstellung von Sonnenschutz-Endformulierungen, enthaltend
(a) 20 bis 35 Gew.% Ölkörper ausgewählt aus linearen und verzweigten Fettalkoholcarbonaten,
(b) 7 bis 17 Gew.% anionisches Tensid ausgewählt aus Alkylpolyalkylenglykolethercitraten,
(c) 3 bis 18 Gew.% weiteres, von (b) verschiedenes Co-Tensid ausgewählt aus Polyol-und/oder Polyglycerinestern;
(d) 35 bis 65 Gew.% mindestens eines öllöslichen UV-Filters; und
(e) 0,01 bis 5 Gew.-% Wasser sowie ggf. 0, 5 bis 5 Gew.-% Hilfsstoffe: unter der Bedingung, dass sich alle Bestandteile auf 100 Gew.-% addieren.
Die erfindungsgemässen Mittel sind lagerstabil und zeigen auch nach längerer Lagerung kein Auskristallisieren bzw. Bodensatz an UV-Filtern. Die Mittel können problemlos mit Wasser versetzt werden, wodurch sehr feinteilige Sonnenschutz-Endformulierungen vom O/W- Emulsionstyp entstehen, die wiederum auch sehr lagerstabil und feinteilig homogen sind. Zudem sind die Mittel im Sinne eines Konzentrats geeignet, dem Anwender freie Gestaltungsmöglichkeiten für die Einstellung des gewünschten SPF-Faktors der Sonnenschutz-Endformulierung zu geben.

Im Sinne der vorliegenden Erfindung dient der SPF-Faktor (*Sun Protection Factor,* SPF) der Beurteilung von Lichtschutzpräparaten (Sonnencremes) am Menschen (in vivo). Er gibt an, wie viel Mal länger man sich mit einem Sonnenschutzmittel der Sonne aussetzen kann, ohne einen Sonnenbrand zu erleiden, als dies mit der jeweils individuellen Eigenschutzzeit möglich wäre.

Der SPF wird nach der "COLIPA International Sun Protection Factor Test Method" bestimmt, (COLIPA, May 2006), wobei nach standardisiertem Auftragen von Lichtschutzpräparaten die Erhöhung der Hautrötungsschwelle (minimale Erythem-Dosis, MED) in Abhängigkeit der Bestrahlungszeit bestimmt wird.

Im Rahmen der vorliegenden Erfindung kommen als Ölkörper (a) beispielsweise Guerbetalkohole auf Basis von Fettalkoholen mit 6 bis 18, vorzugsweise 8 bis 10 Kohlenstoffatomen (z. B. Eutanol® G), Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat in Betracht. Daneben eignen sich Ester von linearen C₆-C₂₂-Fettsäuren mit verzweigten Alkoholen, insbesondere 2-Ethylhexanol, Ester von C₃-C₃₈-Alkylhydroxycarbonsäuren mit linearen oder verzweigten C₆-C₂₂-Fettalkoholen, insbesondere Diethylhexylmalat, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis von C₆-C₁₀-Fettsäuren, flüssige Mono-/Di-Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C-Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen (Hydagen® HSP, Sovermol® 750, Sovermol® 1102), Siliconöle (Cyclomethicone, Siliciummethicontypen u.a.) und/oder aliphatische bzw. naphthenische Kohlenwasserstoffe, wie z.B. Mineralöl, Vaseline, Petrolatum, Squalan, Squalen, Isohexadecane oder Dialkylcyclohexane in Betracht.

Bevorzugte Ölkörper (a) sind mittelpolare Öle, insbesondere Ester von C₂-C₁₂-Dicarbonsäuren mit linearen oder verzweigten Alkoholen mit 1 bis 22 Kohlenstoffatomen und/oder lineare und verzweigte C₆-C₂₂-Fettalkoholcarbonate. Hierbei sind Adipinsäureester von linearen oder verzweigten Alkoholen mit 1 bis 22 Kohlenstoffatomen, ganz besonders von linearen Alkoholen mit 1 bis 6 Kohlenstoffatomen besonders geeignet.

Besonders bevorzugt werden als Ölkörper (a) lineare und verzweigte Fettalkoholcarbonate, insbesondere Dicaprylyl Carbonate verwendet.

Hervorragende Ergebnisse werden mit Dibutyladipat erzielt.

Vorzugsweise sind die Ölkörper in einer Menge von 20 - 35 Gew.-%, bezogen auf die erfindungsgemässen Mittel, enthalten.

Die anionischen Tenside (b) sind Alk(en)ylpolyglykolethercitrate und insbesondere Mischungen von Mono-, Di- und Triestern der Zitronensäure und alkoxylierten Alkoholen, die der Formel (I) entsprechen: 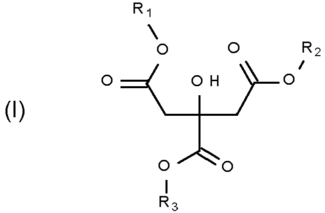 worin
R₁, R₂ und R₃ unabhängig voneinander für Wasserstoff oder den Rest der Formel (II) R₄(OCH₂CHR₅)ₙ
bedeuten, worin
R₄ für einen linearen oder verzweigten Alkyl- und/oder Alkenylrest mit 6 bis 22 Kohlenstoffatomen,
R₅ für Wasserstoff oder Methylrest und
n für eine Zahl von 1 bis 20 stehen, mit der Bedingung, dass zumindest einer der Reste R₁, R₂, oder R₃ verschieden von Wasserstoff ist.

Typische Beispiele für den Alkoholteil der Ester sind Additionsprodukte von durchschnittlich 1 bis 20 Mol, vorzugsweise 5 bis 10 Mol Ethylenoxid und/oder Propylenoxid an Capronalkolohol, Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkcohol, Erucylalkohol and Brassidylalkohol und technische Mischungen davon.

Es ist besonders bevorzugt, Alkylpolyalkylenglykolethercitrate zu verwenden, basierend auf Additionsprodukten von 5 bis 10, insbesondere ungefähr 7 Mol Ethylenoxid an technischen C₁₂-C₁₈, insbesondere C₁₂-C₁₄ Fettalkoholfraktionen. Ganz besonders bevorzugt sind die Polyethylenglykolether des Laurylalkohol, Laureth-7 citrate, die beispielsweise unter dem Namen Plantapon® LC7 (BASF & Personal Care & Nutrition GmbH) erhältlich sind.

Derartige Alk(en)ylpolyglykolethercitrate sind vorteilhaft für die erfindungsgemässen Mittel, da sie flüssige anionische Tenside darstellen mit einem niedrigen Wassergehalt von max. 5 Gew.-%, bezogen auf das anionische Tensid.

Die anionischen Tenside (b) sind vorzugsweise in Mengen von 7 bis 17 Gew.-%, bezogen auf die erfindungsgemässen Mittel, enthalten.

Weiterhin sind in den erfindungsgemässen Mitteln mindestens (c) 0,5 bis 25 Gew.-% eines weiteren von (b) verschiedenen Co-Tensids enthalten.

Als Co-Tenside sind prinzipiell zwitterionische, ampholytische, kationische und/oder nichtionische Tenside geeignet.

Als zwitterionische Tenside werden solche oberflächenaktiven Verbindungen bezeichnet, die im Molekül mindestens eine quartäre Ammoniumgruppe und mindestens eine -COO(-)- oder -SO₃(-)-Grupp tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. Erfindunsggemäss besonders bevorzugt ist Tego® Betain 810 (INCI: Capryl/Capramidopropyl Betaine) sowie eine Tensidmischung aus Rewopol® SBCS 50K (INCI: Disodium PEG-5 Laurylcitrate Sulfosuccinate, Sodium Laureth Sulfate) und Tego® Betain 810 (Capryl/Capramidopropyl Betaine), insbesondere im Gewichtsverhältnis 1:4 bis 4:1, ganz besonders bevorzugt im Gewichtsverhältnis von 1:4 bis 1:1.

Unter ampholytischen Tensiden werden solche oberflächenaktiven Verbindungen verstanden, die ausser einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Bevorzugte ampholytische Tenside sind das N-Kokosalkylaminopropionat, das Kokosacylaminoethylaminopropionat und das C₁₂₋₁₈-Acylsarcosin.

Als kationische Tenside sind insbesondere quartäre Ammoniumverbindungen verwendbar. Tenside aus dieser Stoffklasse haben eine besonders hohe Affinität zur Haut und können den Grad der sensorischen Glätte verbessern. Hierzu zählen unter anderem Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretriethanolaminestersalze verstanden. Sie verleihen den Zusammensetzungen einen besonderen Weichgriff. Es handelt sich dabei um bekannte Stoffe, die man nach den einschlägigen Methoden der organischen Chemie herstellt. Weitere erfindungsgemäss verwendbare kationische Tenside stellen die quaternisierten Proteinhydrolysate dar.

Insbesondere bevorzugt sind als Co-Tenside nichtionische Tenside enthalten, beispielsweise
- Anlagerungsprodukte von 2 bis 50 Mol Ethylenoxid und/oder 0 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe;
- C_{12/18}-Fettsäuremono- und -diester von Anlagerungsprodukten von 1 bis 50 Mol Ethylenoxid an Glycerin;Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- Alkylmono- und -oligoglycoside mit 8 bis 22 Kohlenstoffatomen im Alkylrest und deren ethoxylierte Analoga;Anlagerungsprodukte von 7 bis 60 Mol Ethylenoxid an Ricinusöl und/oder gehärtetes Ricinusöl;
- Polyol- und/oder Polyglycerinester, wie z. B. Polyglycerindiisostearat oder Polyglycerindimerat oder Polyglycerin-12 Hydroxystearat;
- Anlagerungsprodukte von 2 bis 15 Mol Ethylenoxid an Ricinusöl und/oder gehärtetes Ricinusöl;
- Partialester auf Basis linearer, verzweigter, ungesättigter bzw. gesättigter C₆-C₂₂-Fettsäuren, Ricinolsäure sowie 12-Hydroxystearinsäure mit Pentaerythrit, Dipentaerythrit, Zuckeralkoholen (z. B. Sorbit), Alkylglucosiden (z. B. Methylglucosid, Butylglucosid, Laurylglucosid) sowie Polyglucosiden (z. B. Cellulose), oder Mischester wie z. B. Glycerylstearatcitrat und Glycerylstearatlactat;
- Wollwachsalkohole;
- Polysiloxan-Polyalkyl-Polyether-Copolymere bzw. entsprechende Derivate;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin; und
- Polyalkylenglykole.

Die Anlagerungsprodukte von Ethylenoxid und/oder von Propylenoxid an Fettalkohole,

Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. Für die erfindungsgemässen Zubereitungen sind die Umsetzungsprodukte mit 1 - 100 Mol Ethylenoxid besonders gut geeignet.

Vorteilhaft aus der Gruppe der nichtionischen Tenside sind Partialester von Polyolen, insbesondere von C₃-C₆-Polyolen, wie beispielsweise Glycerylmonoestern, Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als nichtionische Tenside geeignet sind auch Anlagerungsprodukte von 1 bis 30, vorzugsweise 5 bis 10 Mol Ethylenoxid an die genannten Sorbitanester.

Nichtionische Tenside aus der Gruppe der Alkyloligoglycoside sind besonders hautfreundlich und können daher im Sinne der Erfindung bevorzugt geeignet sein. C₈-C₂₂-Alkylmono- und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen, vorzugsweise 12 bis 22, und besonders bevorzugt 12 bis 18 C-Atomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare® zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nichtionische Tenside geeignet.

Ganz besonders bevorzugt sind als Co-Tenside nichtionische Tenside, vorzugsweise Polyol- und/oder Polyglycerinester in den erfindungsgemässen Mitteln als Komponente (c) enthalten und / oder Alkyloligoglycoside.

Die Polyolkomponente dieser Tenside kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
- Glycerin und Polyglycerin;
- Alkylenglycole, wie beispielsweise Ethylenglycol, Diethylenglycol, Propylenglycol;
- Methylolverbindungen, wie insbesondere Trimethylolethan, Trimethylolpropan, Trimethylolbutan, Pentaerythrit und Dipentaerythrit;
- Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit;
- Zucker mit 5 bis 12 Kohlenstoffatomen, wie beispielsweise Glucose oder Saccharose;
- Aminozucker, wie beispielsweise Glucamin.

Umsetzungsprodukten auf Basis von Polyglycerin kommt wegen ihrer ausgezeichneten anwendungstechnischen Eigenschaften eine besondere Bedeutung zu.

Die Säurekomponente dieser Tenside kann sich von geradkettigen, verzweigten, gesättigten und/oder ungesättigten Carbonsäuren ggf. mit funktionellen Gruppen wie Hydroxylgruppen ableiten. Besonders bevorzugt handelt es sich bei der Säurekomponente um Fettsäuren mit 12 bis 22 Kohlenstoffatomen, die ggf. eine Hydroxygruppe tragen und insbesondere um Hydroxystearinsäure.

Als besonders vorteilhaft haben sich als nichtionische Tenside Umsetzungsprodukte von Poly-12-hydroxystearinsäure mit Polyglycerinen folgender Homologenverteilung erwiesen (bevorzugte Mengen sind in Klammern angegeben):

| | |
|---|---|
| Glycerine: | 5 bis 35 (15 bis 30) Gew.-% |
| Diglycerine: | 15 bis 40 (20 bis 32) Gew.-% |
| Triglycerine: | 10 bis 35 (15 bis 25) Gew.-% |
| Tetraglycerine: | 5 bis 20 (8 bis 15) Gew.-% |
| Pentaglycerine: | 2 bis 10 (3 bis 8) Gew.-% |
| Oligoglycerine: | ad 100 Gew.-% |

In einer bevorzugten Ausführungsform der Erfindung wird als Glycerylester der Diester der Polyhydroxystearinsäure, Polyglyceryl-2 Dipolyhydroxystearate, verwendet, der beispielsweise von der BASF Personal Care and Nutrition GmbH unter der Bezeichnung Dehymuls® PGPH vermarktet wird.

In den erfindungsgemässen Mitteln sind die weiteren Co-Tenside üblicherweise in einer Menge von 0,5 - 25 Gew.-% vorhanden; bevorzugt ist eine Menge von 3,0 - 18 Gew.-% und insbesondere 7 - 18 Gew.-%.

Schliesslich enthalten die erfindungsgemässen Mittel hohe Mengen, d.h. im Bereich von 25 bis 75 Gew.%, vorzugsweise 35 bis 65 Gew.%, insbesondere 40 bis 60 Gew.% an UV-Filtern (d).

Als UV Filter entsprechend der Komponente (d) werden erfindungsgemäss die folgenden Substanzlassen vorzugsweise eingesetzt (INCI-Bezeichnungen):
- (d₁): p-Aminobenzoesäurederivative,
- (d₂): Salicylsäurederivative,
- (d₃): Benzophenonderivate,
- (d₄): Dibenzoylmethanederivative,
- (d₅): Diphenylacrylate,
- (d₆): 3-Imidazol-4-yl-Acrylsäure und ihre Ester;
- (d₇): Benzofuranderivative;
- (d₈): polymere UV absorber;
- (d₉): Zimtsäurederivate;
- (d₁₀): Campherderivative;
- (d₁₁): Hydroxyphenyltriazinderivate;
- (d₁₂): Benzotriazolderivate;
- (d₁₃): Trianilino-s-triazinderivate;
- (d₁₄): 2-Phenylbenzimidazol-5-sulfonsäure und deren Salze;
- (d₁₅).: Menthyl o-aminobenzoate;
- (d₁₆): Homosalate;
- (d₁₇): Tris-biphenyl-triazinderivate;
- (d₁₈): TiO₂ (zum Teil enkapsuliert), ZnO und Mica;
- (d₁₉): Benzylidenmalonate;
- (d₂₀): Merocyaninderivate;
- (d₂₁): Phenylene bis diphenyltriazine;
- (d₂₂): Imidazolinederivate; und
- (d₂₃): Diarylbutadienderivate.

Beispielhaft einsetzbare Verbindungen für p-Aminobenzoesäurederivate (d₁) sind 4-Aminobenzoesäure (PABA); Ethyldihydroxypropyl-PABA der Formel 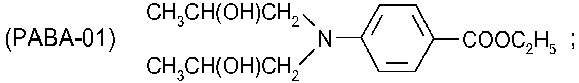 PEG-25-PABA der Formel 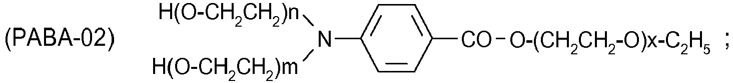 worin m, n und x dieselbe Bedeutung haben und je höchstens 25 bedeuten; Octyldimethyl PABA der Formel 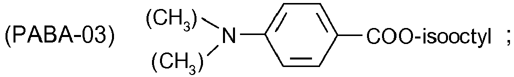 oder Glycylaminobenzoat der Formel 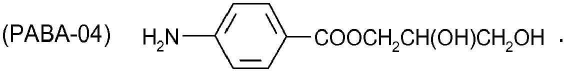 Beispielhaft einsetzbare Verbindungen für Salicylsäurederivate (d₂) sind Homomenthylsalicylat der Formel 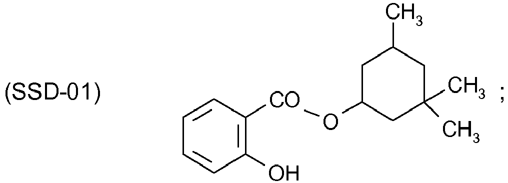 Triethanolaminsalicylat der Formel 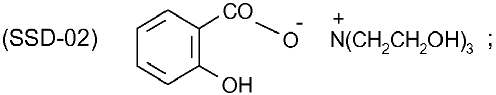 Amyl-p-dimethylaminobenzoat der Formel 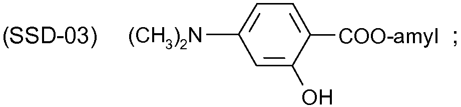 Octylsalicylat der Formel 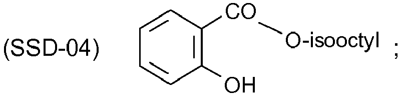 oder 4-Isopropylbenzylsalicylat der Formel 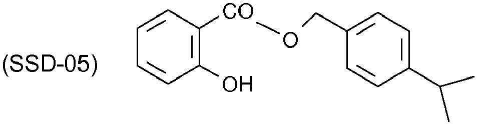 Beispielhaft einsetzbare Verbindungen für Benzophenonderivate (d₃) sind:
Benzophenon-3-(2-hydroxy-4-methoxybenzophenon); Benzophenon-4-(2-hydroxy-4-methoxybenzophenon-5-sulfonsäure); Benzophenon-8-(2,2'-dihydroxy-4-methoxybenzo-phenon); oder aminosubstituierte Hydroxybenzophenone der Formel 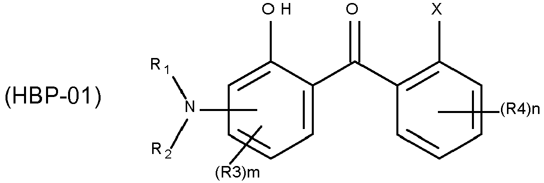 worin
   R₁ und R₂ Wasserstoff, C₁-C₂₀-Alkyl, C₂-C₁₀-Alkenyl, C₃C₁₀-Cycloalkyl, C₃-C₁₀-Cycloalkenyl, wobei die Substituenten R₁ und R₂ gemeinsam mit dem Stickstoffatom, an den sie gebunden sind, einen 5- oder 6-Ring bilden können;
   R₃ und R₄, unabhängig voneinander C₁-C₂₀-Alkyl; C₂-C₁₀-Alkenyl; C₃-C₁₀-Cycloalkyl; C₃-C₁₀-Cycloalkenyl; C₁-C₂₂-Alkoxy; C₁-C₂₀-Alkoxycarbonyl; C₁-C₁₂-Alkylamino; C₁-C₁₂-Dial-kylamino; gegebenenfalls substituiertes Aryl; Heteroaryl; wasserlöslich machende Substituenten, ausgewählt aus der Gruppe bestehend aus einer Nitrilgruppe, Carboxylat-, Sultonat- oder Ammoniumresten;
   X Wasserstoff; COOR₅; oder CONR₆R₇;
   R₅, R₆, R₇, unabhängig voneinander, Wasserstoff; C₁-C₂₀-Alkyl; C₂-C₁₀-Alkenyl; C₃-C₁₀-Cycloalkyl; C₃-C₁₀-Cycloalkenyl; (Y-O)ₒ-Z; oder Aryl;
   Z -CH₂-CH₃; -CH₂-CH₂-CH₃; -CH₂-CH₂-CH₂-CH₃; oder -CH(CH₃)-CH₃;
   m 0 bis 3 ;
   n 0 bis 4 ; und
   o 1 bis 20;
   bedeuten.

Besonders bevorzugt ist Diethylamino Hydroxybenzoyl Hexyl Benzoate entsprechend der Formel

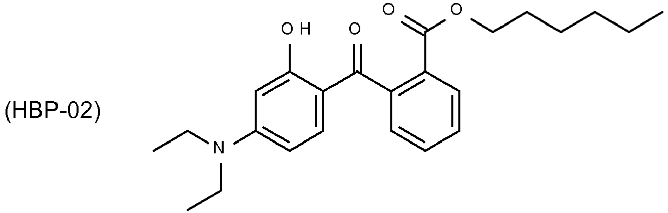

Erfindungsgemäss einsetzbar sind auch dimere Benzophenonderivate entsprechend der Formel 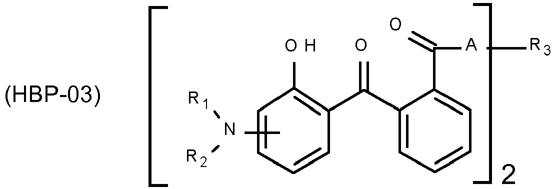 worin
R₁ und R₂ unabhängig voneinander C₁-C₂₀-Alkyl; C₂-C₂₀-Alkenyl; C₃-C₁₀-Cycloalkyl; C₃-C₁₀-Cycloalkenyl; oder R₁ und R₂ zusammen mit dem Stickstoffatom, an das sie gebunden sind, einen 5- oder 6-gleidrigend heterocyclischen Ring bilden;
R₃ gegebenenfalls mit einer Carbonyl- oder Carboxy-Gruppe substituiertes Alkylen-, Cycloalkylen, Alkenylen oder Phenylen, ; ein Biradikal der Formel (HBP-03a) *-CH₂-C≡C-CH₂-* ; oder R₃ zusammen mit A einen bivalenten Rest der Formel 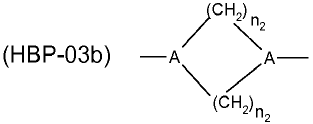 bildet, worin
n₂ eine Zahl von 1 bis 3;
A -O-; oder-N(R₅)-; und
R₅ Wasserstoff; C₁-C₅alkyl; oder Hydroxy-C₁-C₅alkyl;
   bedeuten.

Insbesondere sind dimere Benzophenonderivate der Formel 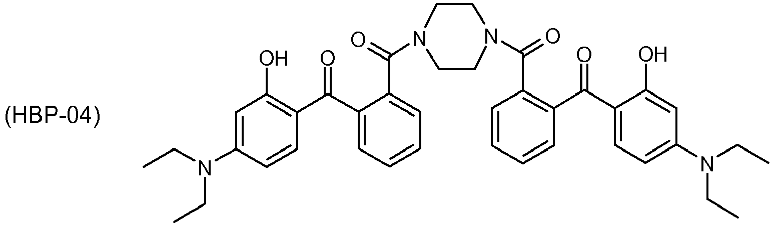 und 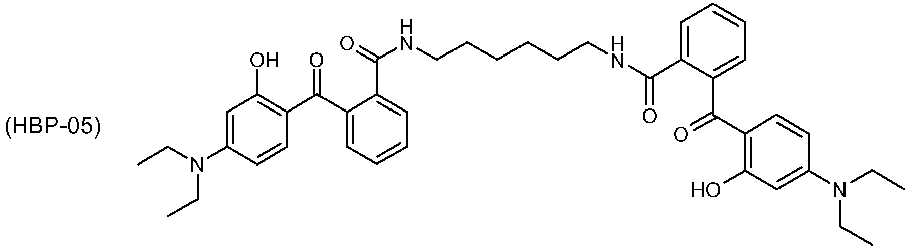 als UV-Filter (d₃) bevorzugt einsetzbar.

Beispielhafte erfindungsgemäss einsetzbare Dibenzoylmethanderivate (d₄) sind Butyl-methoxydibenzoylmethan-[1-(4-tert.-butyl)-3-(4-methoxyphenyl)propan-1,3-dion].

Beispielhafte erfindungsgemäss einsetzbare Diphenylacrylatderivate (d₅) sind Octocrylen-(2-ethylhexyl-2-cyano-3,3'-diphenylacrylat) oder Etocrylen-(ethyl-2-cyano-3,3'-diphenylacrylat).

Beispielhafte erfindungsgemäss einsetzbare Benzofuranderivate (d₇) sind 3-(Benzofuranyl)-2-cyanoacrylat, 2-(2-Benzofuranyl)-5-tert.-butylbenzoxazol oder 2-(p-Aminophenyl)benzofuran und insbesondere die Verbindungen der Formel 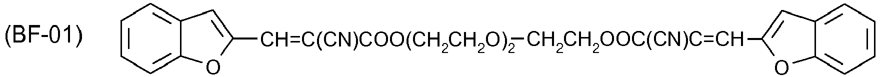 oder

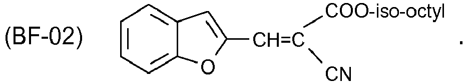

Beispielhafte erfindungsgemäss einsetzbare polymere UV-Absorber (d₈), die eine oder mehrere silizium-organische Reste enthalten, sind Benzylidenmalonatderivate, insbesondere die Verbindung der Formel 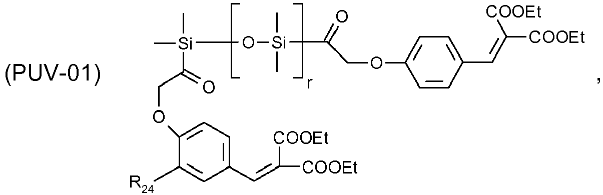 worin R₂₄ Wasserstoff oder

Methoxy und r ungefähr 7 bedeuten; die Verbindung der Formel 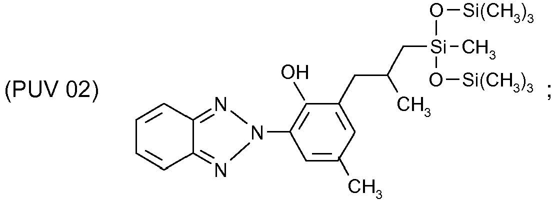 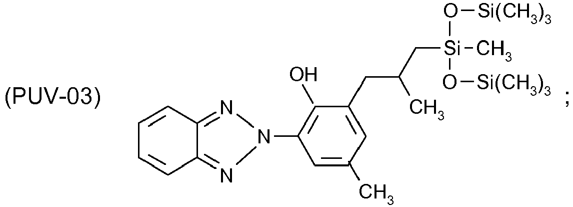 oder Poliyisilicone-15 entsprechend der Formel

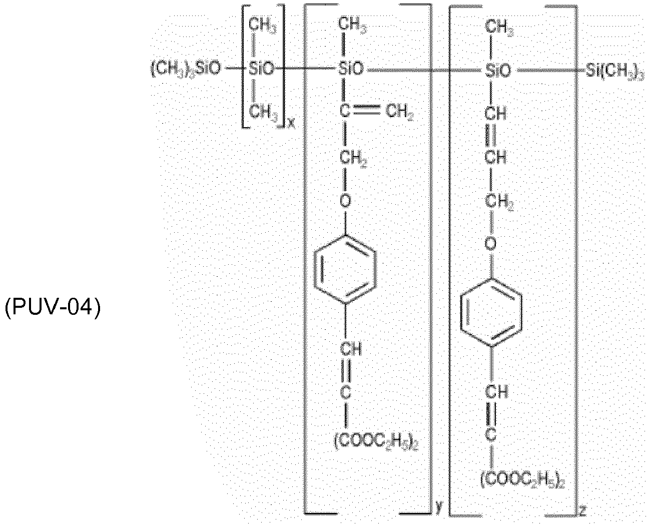

Beispielhafte erfindungsgemäss einsetzbare Zimtsäureester (d₉) sind Octylmethoxycinnamat (4-Methoxyzimtsäure-2-ethylhexylester), Diethanolaminmethoxycinnamat (Diethanolaminsalz der 4-Methoxyzimtsäure), Isoamyl p-methoxycinnamat (4-Ethoxyzimtsäure-2-isoamylester), 2,5-Diisopropylmethylcinnamat oder ein Zimtsäureamidoderivat.

Beispielhafte erfindungsgemäss verwendbare Campherderivate (d₁₀) sind 4-Methyl-benzylidencampher [3-(4'-Methyl)benzyliden-bornan-2-on], 3-Benzylidencampher (3-Benzyliden-bornan-2-on), Polyacrylamidomethylbenzylidencampher {N-[2(und 4)-2-oxyborn-3-yliden-methyl)benzyl]acrylamidpolymer}, Trimonium-benzylidencamphersulfat-[3-(4'-trimethylammonium)-benzyliden-bornan-2-on-methylsulfat], Terephthalydendicampher-Sulfonsäure {3,3'-(1,4-Phenylendimethin)-bis-(7,7-dimethyl-2-oxo-bicyclo-[2.2.1]heptan-1-methansulfonsäure} oder deren Salze, oder Benzylidencampher-Sulfonsäure [3-(4'-sulfo)benzylidenbornan-2-on] oder deren Salze.

Beispielhafte erfindungsgemäss verwendbare Hydroxyphenyltriazinderivate (d₁₁) sind insbesondere Bis-Resorcinyl-Triazine der Formel 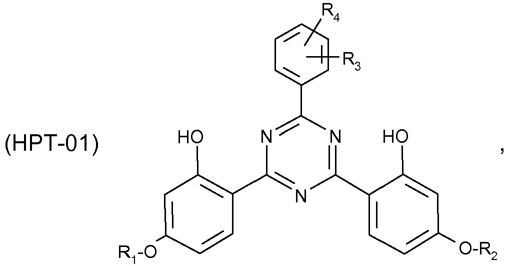 worin
R₁ und R₂, unabhängig voneinander, Wasserstoff; C₁-C₁₈-Alkyl; C₂-C₁₈-Alkenyl; einen Rest der Formel -CH₂-CH(-OH)-CH₂-O-T₁ ;einen Rest der Formel 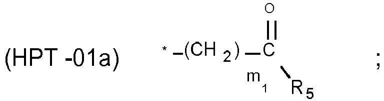 oder einen Rest der Formel (HPT-01h) 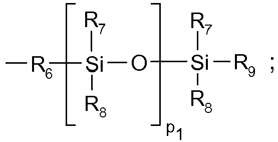
R₃, R₅ und R₅, unabhängig voneinander Hydroxy; nicht substituiertes oder durch eine oder mehrere OH-Gruppen substituiertes C₁-C₅-Alkoxy; Amino; Mono- oder Di-C₁-C₅-Alkylamino; M; einen Rest der Formel 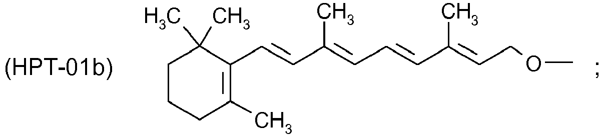 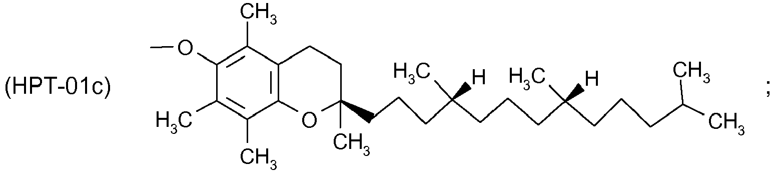 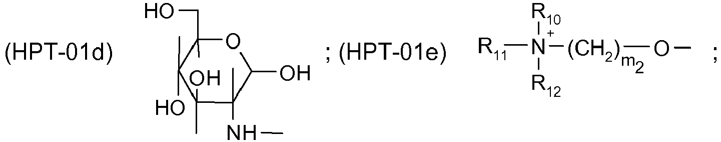 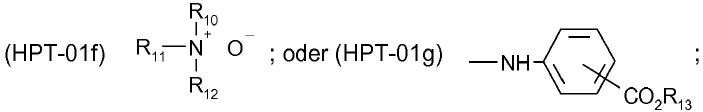
R₁₀, R₁₁ und R₁₂ unabhängig voneinander nicht substituiertes oder durch ein oder mehrere OH-Gruppen substituiertes C₁-C₁₄-Alkyl;
R₁₃ Wasserstoff; M; C₁-C₅-Alkyl; oder einen Rest der Formel -(CH₂)ₘ₃-O-T₁
R₆ die direkte Bindung; einen geradkettigen oder verzweigten C₁-C₄-Alkylenrest; oder einen Rest der Formel -Cₘ₄H₂ₘ₄, oder -Cₘ₄H₂ₘ₄-O-;;
R₇, R₈ und R₉, unabhängig voneinander, C₁-C₁₈-Alkyl; C₁-C₁₈-Alkoxy oder einen Rest der Formel 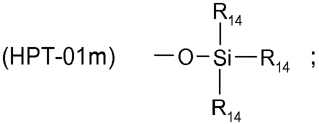
R₁₄ C₁-C₅-Alkyl;
M ein Metallkation;
T₁ Wasserstoff; oder C₁-C₈-Alkyl;
m₁, m₂ und m₃, unabhängig voneinander, 1 bis 3;
m₄ 2 bis 14; und
p₁ 0 oder eine Zahl von 1 bis 5;
bedeuten.

Als beispielhafte Vertreter für die Verbindungsklasse (d₁₁) seien genannt:
- 2-(4'-Methoxyphenyl)-4,6-bis(2'-hydroxy-4'-n-octyloxyphenyl)-1,3,5-triazin;
- 2,4-Bis-{[4-(3-(2-propyloxy)-2-hydroxy-propyloxy)-2-hydroxy]-phenyl}-6-(4-methoxyphenyl)-1,3,5-triazin;
- 2,4-Bis-{[4-(2-ethyl-hexyloxy)-2-hydroxy]-phenyl}-6-[4-(2-methoxyethyl-carboxyl)-phenyl-amino]-1,3,5-triazin;
- 2,4-Bis-{[4-(tris(trimethylsiloxy-silylpropyloxy)-2-hydroxy]-phenyl}-6-(4-methoxyphenyl)-1,3,5-triazin;
- 2,4-Bis-{[4-(2"methylpropenyloxy)-2-hydroxy]-phenyl}-6-(4-methoxyphenyl)-1,3,5-triazin;
- 2,4-Bis-{[4-(1',1',1',3',5',5',5'-Heptamethyltrisilyl-2"-methyl-propyloxy)-2-hydroxy]-phenyl}-6-(4-methoxyphenyl)-1,3,5-triazin;
- 2,4-Bis-{[4-(3-(2-propyloxy)-2-hydroxy-propyloxy)-2-hydroxy]-phenyl}-6-[4-ethylcarboxyl)-phenylamino]-1,3,5-triazin;
- 2,4-Bis-{[4-(2-ethyl-hexyloxy)-2-hydroxy]-phenyl}-6-(1-methylpyrrol-2-yl)-1,3,5-triazin; oder
- 2,2'-[6-(4-Methoxyphenyl)-1,3,5-Triazine-2,4-Diyl]Bis[5-[(2-Ethylhexyl)Oxy]-(Bis-Ethylhexyloxyphenol Methoxyphenyl Triazine) entsprechend der Formel

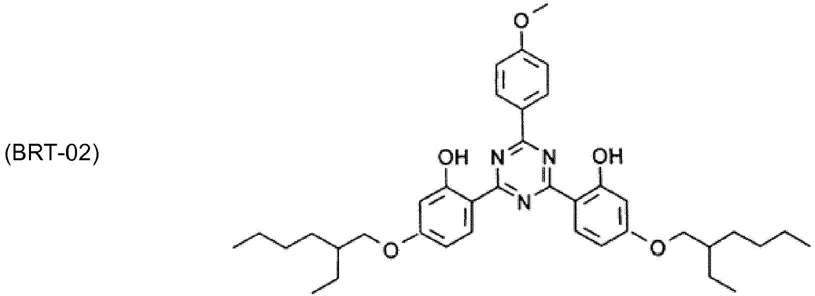

Beispielhafte erfindungsgemäss verwendbare Benzotriazolderivate (d₁₂) entsprechen der Formel 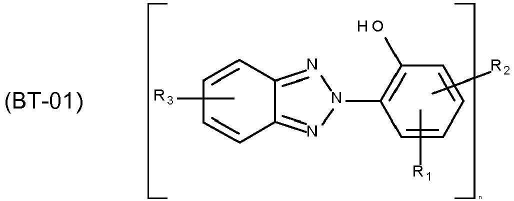 worin
R₁ Wasserstoff; C₁-C₁₂-Alkyl; C₁-C₁₂-Alkoxy; C₁-C₁₂-Alkoxycarbonyl; C5-C₁₀cycloalkyl _{oder}-S0₃M;
R₃ Wasserstoff; C₁-C₁₈-Alkyl; C₁-C₁₂alkoxy; oder Halogen; und
n 1 oder 2; bedeuten;
wenn n = 1 ist, bedeutet
R₂ C₁-C₂₀alkyl; C₅-C₁₀-Cyclo-C₁-C₅-Alkyl; C₁-C₁₂-Alkoxy-C₁-C₅-Alkyl; C₅-C₁₀-Cycloalkoxy-C₁-C₅-Alkyl; C₆-C₁₀-Aryl; C₆-C₁₀-Aryl-C₁-C₅-Alkyl;
wenn n = 2 ist, bedeutet
R₂ die direkte Bindung; oder -(CH₂)ₚ-; und
p ist eine ganze Zahl von 1 bis 3.

Vorzugsweise kommen Verbindungen der Formel (BT-01) in Betracht, worin
R₁ C₁-C₁₂-Alkyl; oder -S0₃M;
R₃ Wasserstoff; Halogen, vorzugsweise Cl;
n 1;
R₂ C₁-C₁₂alkyl; und
p 1 bis 3;
bedeuten.

Ganz besonders bevorzugt sind Verbindungen der Formel

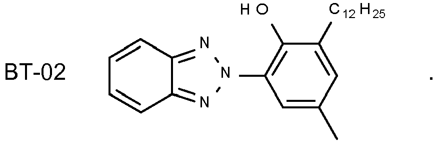

Weiterhin sind UV-Filter der Formel BT-01 bevorzugt, worin
R₁ Wasserstoff;
R₃ C₁-C₁₈-Alkyl;
n = 2; und
R₂ -CH₂-;
bedeuten.

Ganz besonders bevorzugt sind Verbindungen der Formel

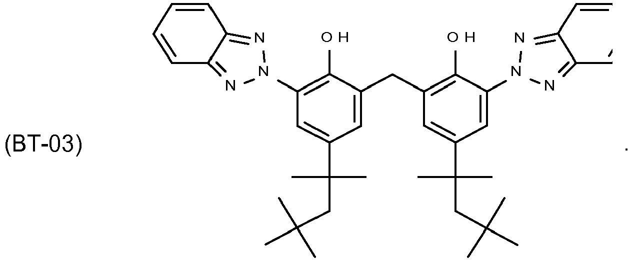

Beispielhafte erfindungsgemäss einsetzbare Trianilino-s-triazinderivate (d₁₃) entsprechen der Formel 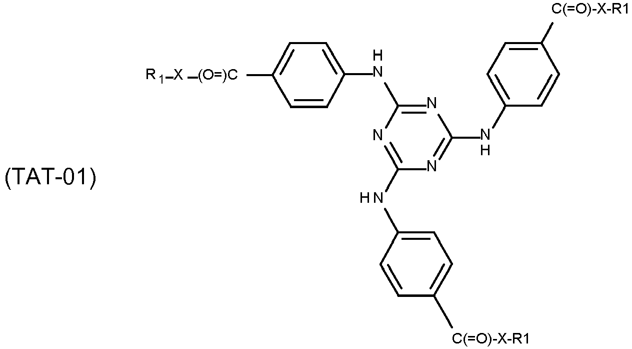 worin
R₁, R₂ und R₃ unabhängig voneinander gegebenenfalls substituiertes C₁-C₂₀-Alkyl, Aryl oder Heteroaryl,;
X O; oder NR₄; und
R₄ Wasserstoff; oder gegebenenfalls substituiertes C₁-C₂₀-Alkyl, Aryl oder Heteroaryl;
bedeuten.

Besonders bevorzugte Vertreter dieser Verbindungsklasse ist Ethylhexyl Triazone entsprechend der Formel 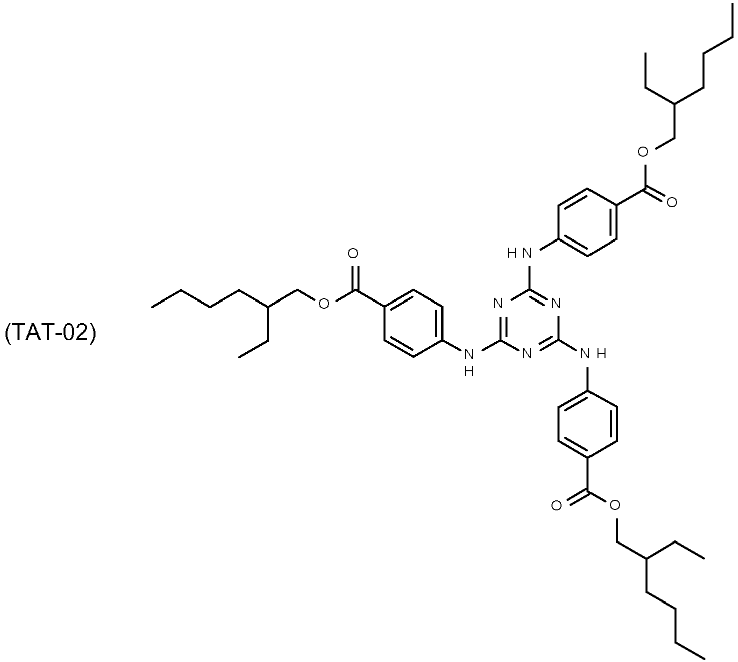 bzw. Diethylhexyl Butamido Triazone entsprechend der Formel 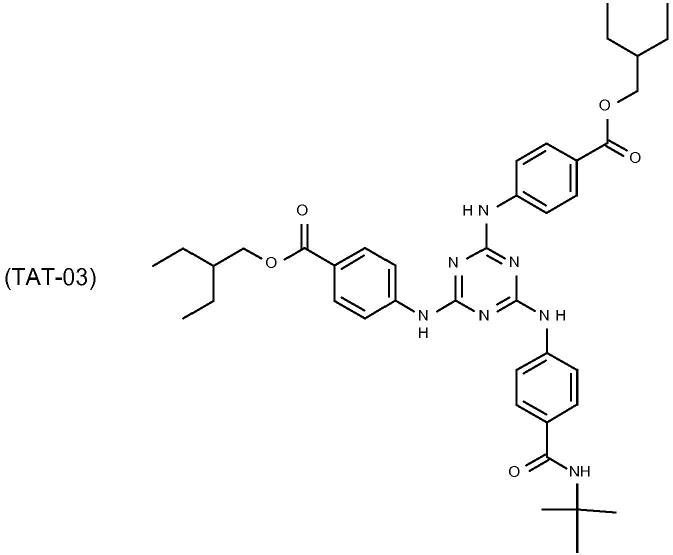 oder Ethylhexyl Bis-Isopentylbenzoxazolylphenyl Melamine entsprechend der Formel

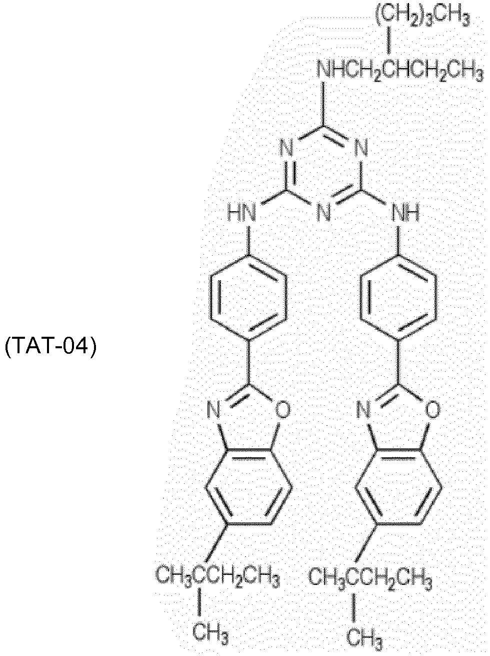

Bevorzugte efindungsgemäss verwendbare Tris-biphenyl-triazinderivate (d₁₇) entsprechen der Formel 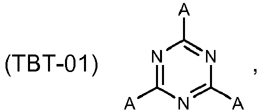 worin
A ein Rest der Formel 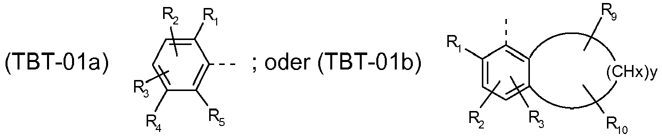
R₁ und R₅ unabhängig voneinander Wasserstoff; C₁-C₁₈-Alkyl; oder C₆-C₁₂-Aryl;
R₂, R₃ und R₄ unabhängig voneinander Wasserstoff; oder ein Rest der Formel 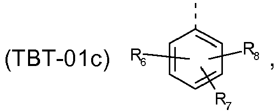 worin, in Formel (TBT-01a), mindestens einer der Reste R₂,
   R₃ und R₄ einen Rest der Formel (TBT-01c) bedeutet;
R₆, R₇, R₈, R₉ und R₁₀ unabhängig voneinander Wasserstoff; Hydroxy; Halogen; C₁-C₁₈-Alkyl; C₁-C₁₈-Alkoxy; C₆-C₁₂-Aryl; Biphenylyl; C₆-C₁₂-Aryloxy; C₁-C₁₈-Alkylthio; Carboxy; -COOM; C₁-C₁₈-Alkylcarboxyl; Aminocarbonyl; oderr mono- or di-C₁-C₁₈-Alkylamino; C₁-C₁₀-Acylamino; -COOH;
M ein Alkalimetallion;
x 1 oder 2; und
y eine ganze Zahl von 2 bis 10;
bedeuten.

Vorzugsweise entsprechen die erfindugunsgemäss verwendbaren UV-Filter (d₁₇) den Verbindungen der Formel 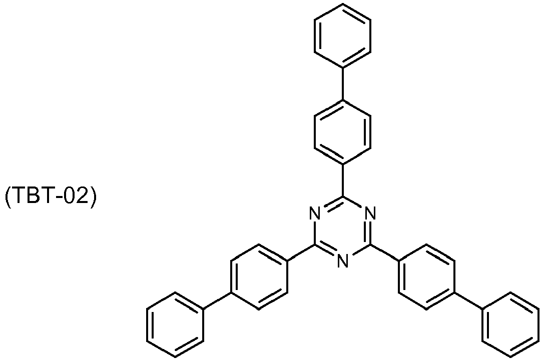 und

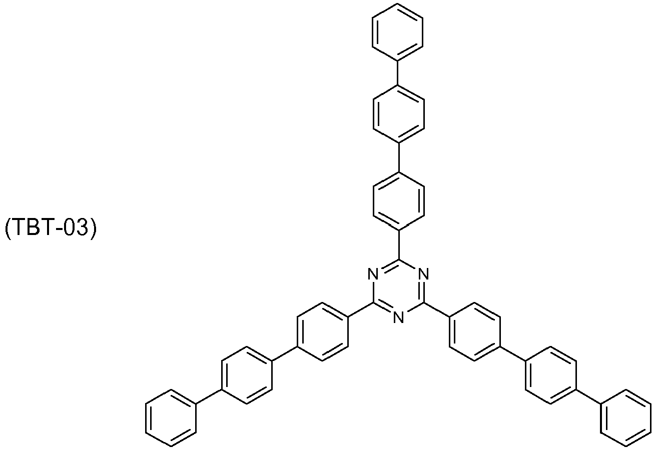

Bevorzugte erfindungsgemäss einsetzbare Benzylidenmalonate (d₁₉) entsprechen der Formel 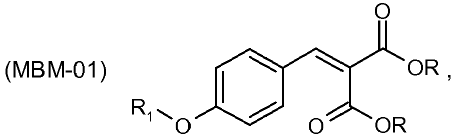 worin
R₁ Methyl; Ethyl; Propyl; oder n-Butyl; bedeuten
wenn R₁ Methyl bedeutet, dann bedeutet
R tert. Butyl; 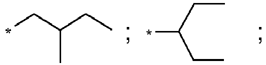 einen Reste der Formel 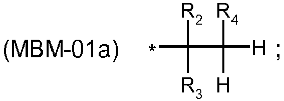 oder einen Rest der Formel (MBM-01b) 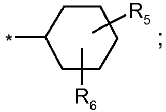 worin
R₂ und R₃, unabhängig voneinander Wasserstoff; oder Methyl;
R₄ Methyl; Ethyl; oder n-Propyl;
R₅ und R₆ unabhängig voneinander Wasserstoff; oder C₁-C₃-Alkyl; Wenn R₁ Ethyl; Propyl; oder n-Butyl bedeuten, dann bedeutet
R Isopropyl.

Besonders bevorzugte erfindungsgemäss einsetzbare Benzylidenmalonate (d₁₉) sind in der folgenden Tabelle aufgeführt:

| Tabelle 1: Beispiele für erfindungsgmäss verwendbarere Benzylidenemalonate | | |
|---|---|---|
| 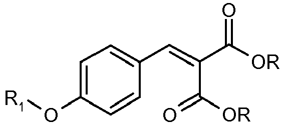 | | |

| | R₁ | R |
|---|---|---|
| (MBM-02) | methyl | 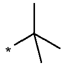 |
| (MBM-03) | methyl | 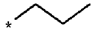 |
| (MBM-04) | methyl | 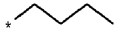 |
| (MBM-05) | methyl | 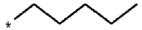 |
| (MBM-06) | methyl | 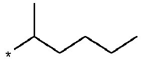 |
| (MBM-07) | methyl | 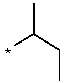 |
| (MBM-08) | methyl | 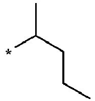 |
| (MBM-09) | methyl | 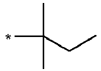 |
| (MBM-10) | methyl | 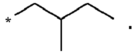 |
| (MBM-11) | ethyl | 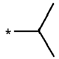 |
| (MBM-12) | propyl | 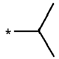 |
| (MBM-13) | n-butyl | 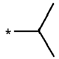 |
| (MBM-14) | methyl | 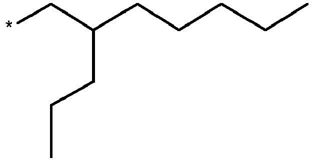 |
| (MBM-15) | methyl | 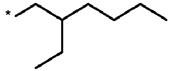 |

Beispielhafter Vertreter für Phenylene-bis-diphenyltriazine (d₂₁) ist 5,6,5,6-tetraphenyl-3,3'-(1,4-phenylene)-bis[1,2,4]-triazin entspricht der Formel

| | | |
|---|---|---|
| (PBT-01) | 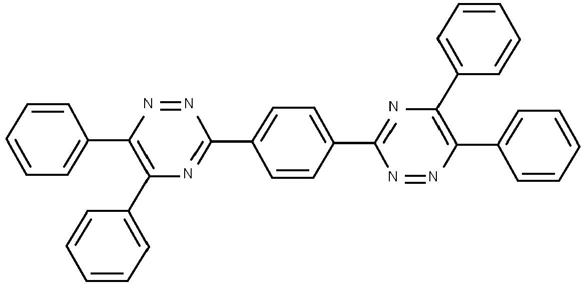 | |

Beispielhafter Vertreter für Imidazolinederivate ist Ethylhexyl-dimethoxybenzylidendioxoimidazolin Propionate.

Beispielhafter Vertreter für Diarylbutadienderivate (d₂₃) ist 1,1-Dicarboxy-(2,2'-dimethylpropyl)-4,4-diphenylbutadien.

Jeder der oben genannten UV-Filter (d₁) - (d₂₃) kann als Mischung erfindungsgemäss verwendet werden. Zum Beispiel können Mischungen aus zwei, drei, vier, fünf oder sechs der Filtergruppen (d₁) - (d₂₃) erfindungsgemäss verwendet werden. Es können auch Mischungen aus zwei, drei, vier, fünf oder sechs UV-Filtern aus einer oder mehreren Vertretern der Stoffklassen (d₁) - (d₂₃) erfindungsgemäss verwendet werden.

Bevorzugt werden erfindungsgemäss UV-Filter (d) verwendet, insbesondere Vertreter der folgenden Verbindungsklassen:
- (d₁): p-Aminobenzoesäurederivative,
- (d₂): Salicylsäurederivative,
- (d₃): Benzophenonderivate,
- (d₄): Dibenzoylmethanederivative,
- (d₅): Diphenylacrylate,
- (d₆): 3-Imidazol-4-yl-Acrylsäure und ihre Ester;
- (d₇): Benzofuranderivative;
- (d₉): Zimtsäurederivate;
- (d₁₀): Campherderivative;
- (d₁₁): Hydroxyphenyltriazinderivate;
- (d₁₂): Benzotriazolderivate;
- (d₁₃): Trianilino-s-triazinderivate;
- (d₁₅).: Menthyl o-aminobenzoate;
- (d₁₆): Homosalate;

- (d₁₉): Benzylidenmalonate; und
- (d₂₀): Merocyaninderivate.

Besonders bevorzugt werden erfindungsgemäss die folgenden öllöslichen UV-Filter verwendet:
- (d_{SOL-1}): Benzophenone-3 (BP3);
- (d_{SOL-2}): Benzophenone-4 (BP4);
- (d_{SOL-3}): 3-Benzydilene Camphor (3BC);
- (d_{SOL-4}): Bis-Ethylhexyloxyphenol Methoxyphenyl Triazine (BEMT);
- (d_{SOL-5}): Butyl Methoxydibenzoylmethane (BMBM);
- (d_{SOL-6}): Diethylhexyl Butamido Triazone (DBT);
- (d_{SOL-7}): Drometrizole Trisiloxane (DTS);
- (d_{SOL-8}): Ethylhexyl Triazone (EHT);
- (d_{SOL-9}): Ethylhexyl Methoxycinnamate;
- (d_{SOL-10}): Benzylidenemalonat (BM);
- (d_{SOL-11}): Diethylamino Hydroxy Benzoyl Hexyl Benzoate (DHHB);
- (d_{SOL-12}): Octocrylen;
- (d_{SOL-13}): Polysilicon1-15;
- (d_{SOL-14}): Homosalat; und
- (d_{SOL-15}): Ethylhexylsalicylat.

Ganz besonders bevorzugt im Sinne der vorliegenden Erfindung sind öllösliche UV-Filtermischungen aus
- (d₉ₐ): Ethylhexyl Methoxycinnamate,
- (d₁₁ₐ): Bis-Ethylhexyloxyphenol Methoxyphenyl Triazine,
- (d₁₃ₐ): Ethylhexyl Triazone und
- (d₃ₐ): Diethylamino Hydroxy Benzoyl Hexyl Benzoate.

In unten stehender Tabelle sind mögliche erfindungsgemässe UV-Filtermischungen aus öllöslichen UV-Filtern (UV SOL 1 - UV SOL 551) aufgeführt:

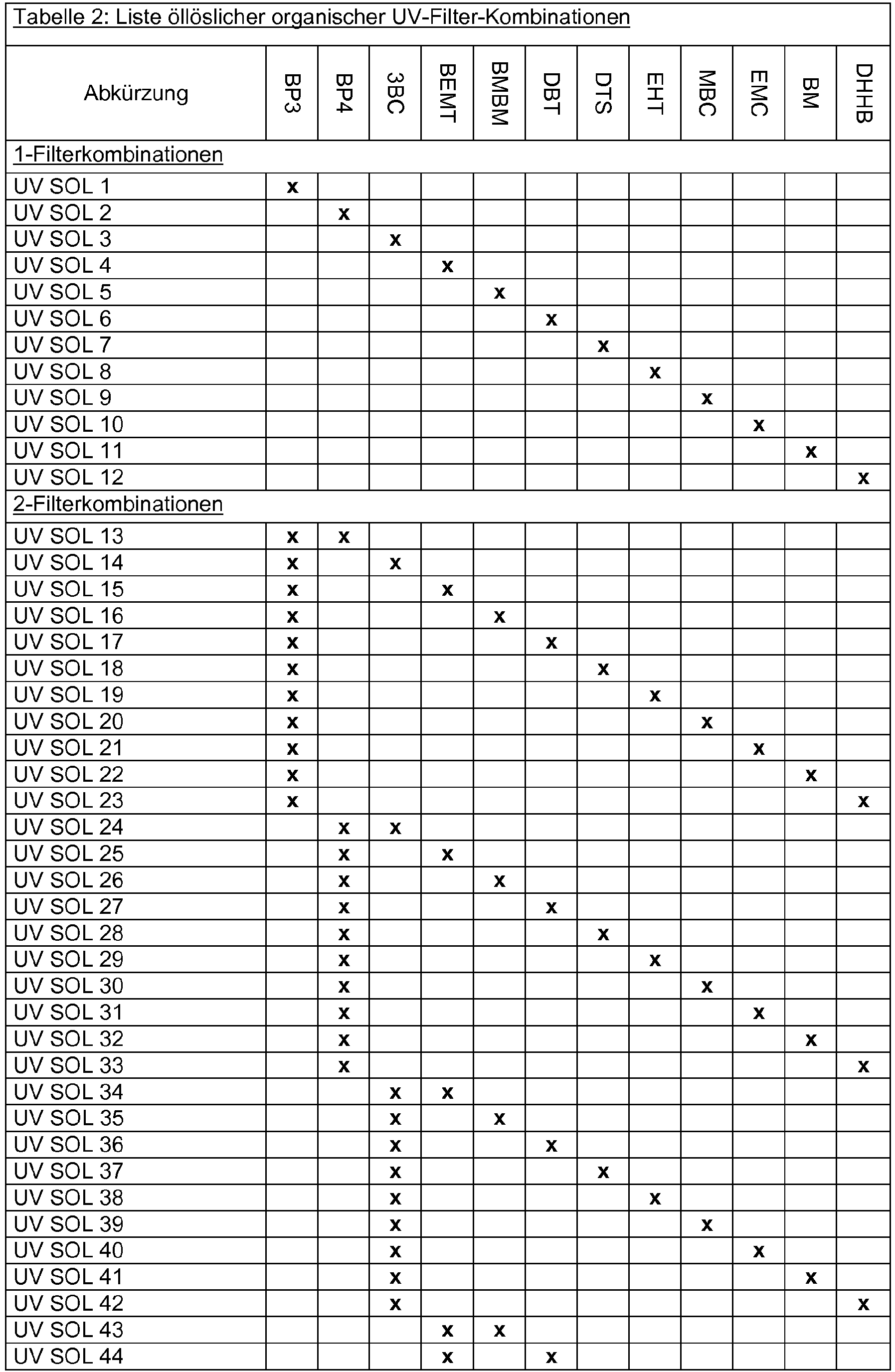

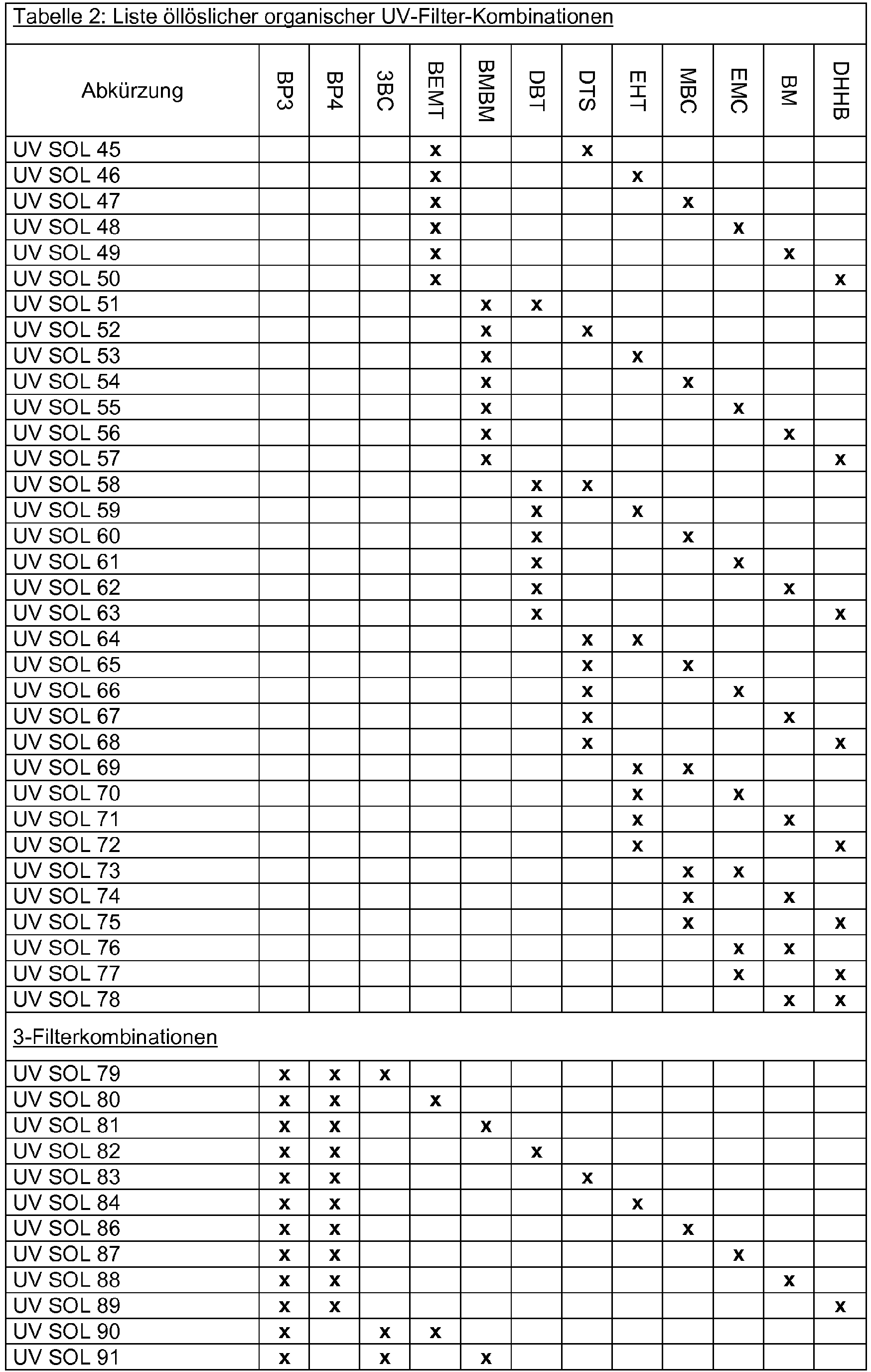

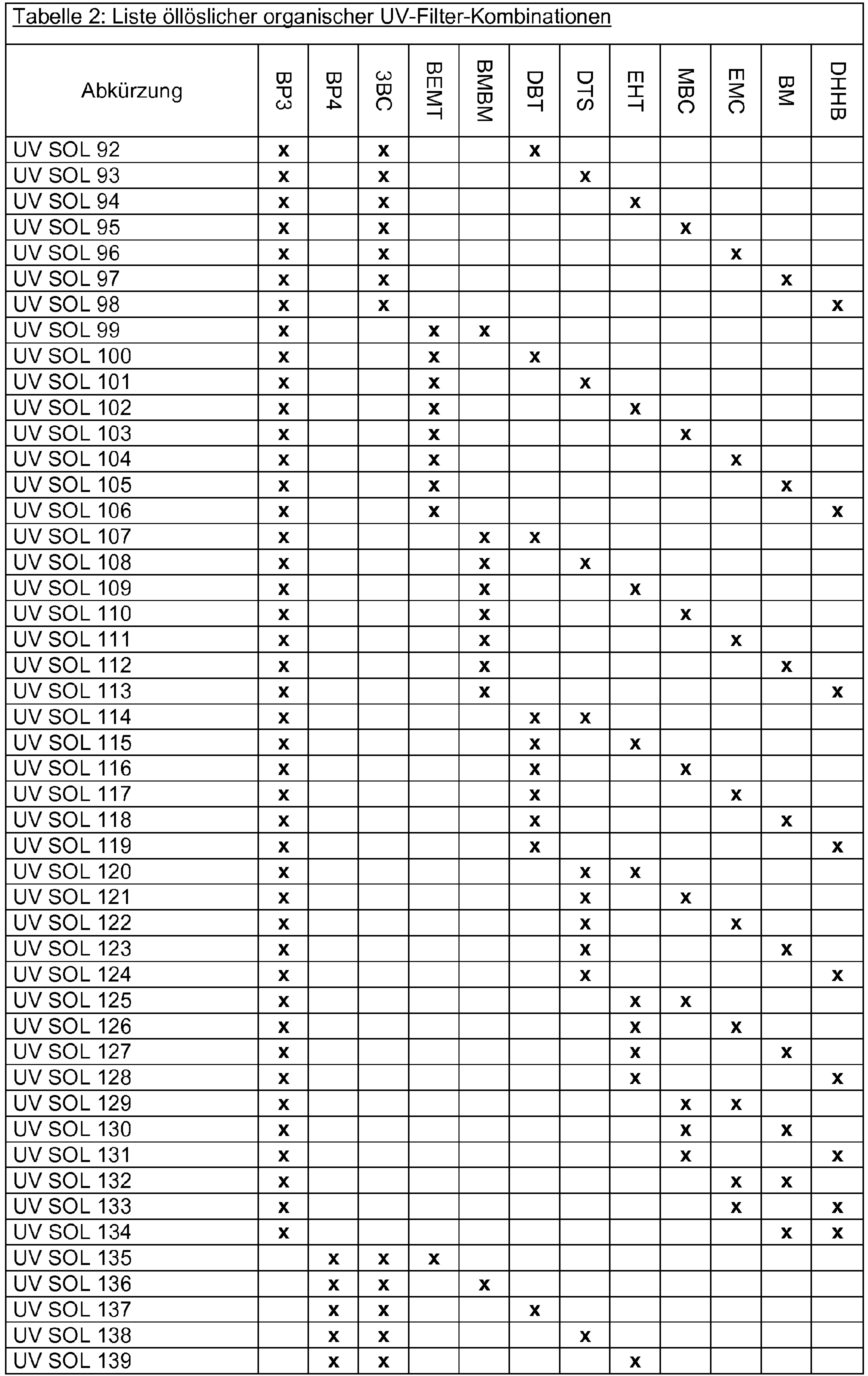

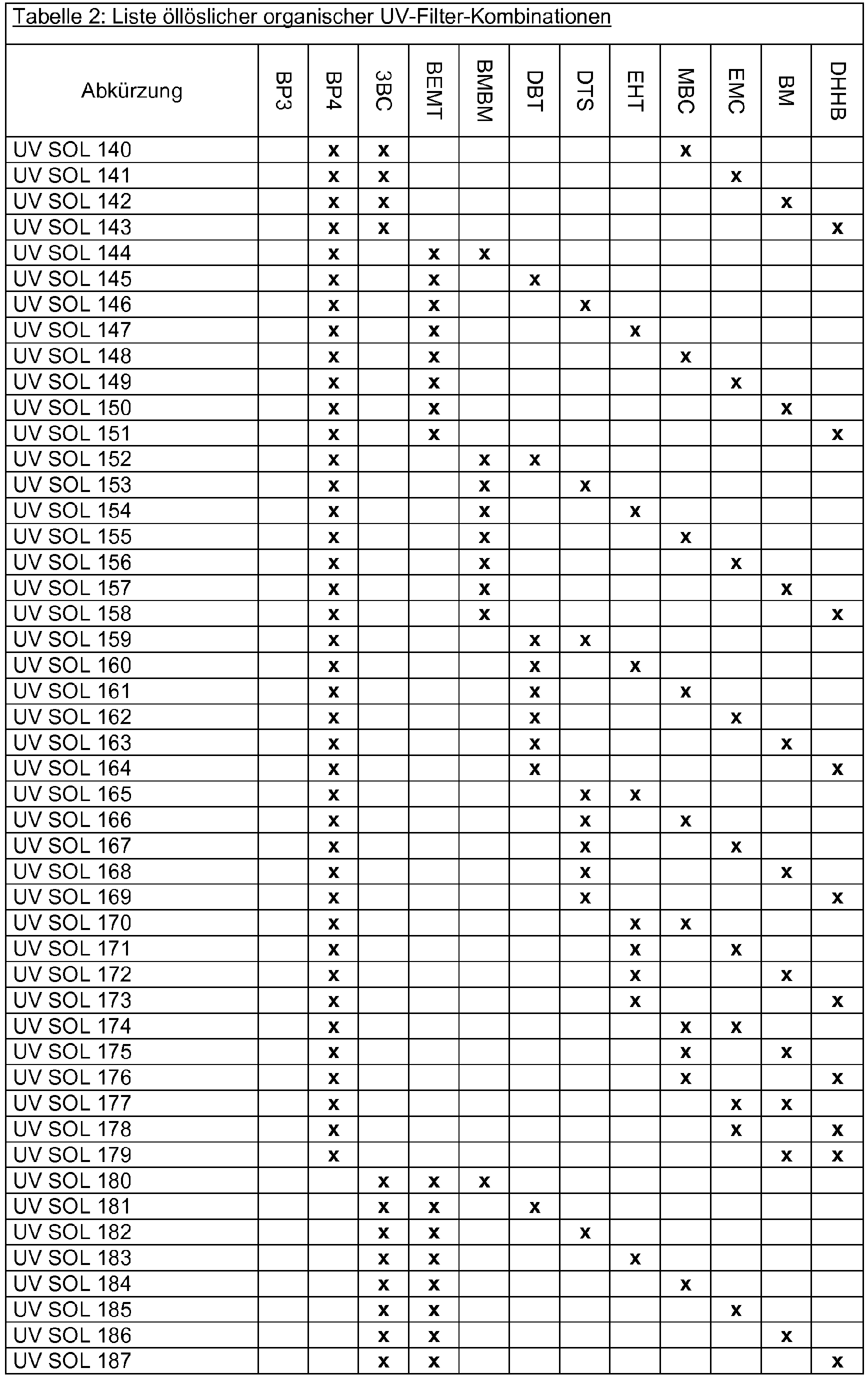

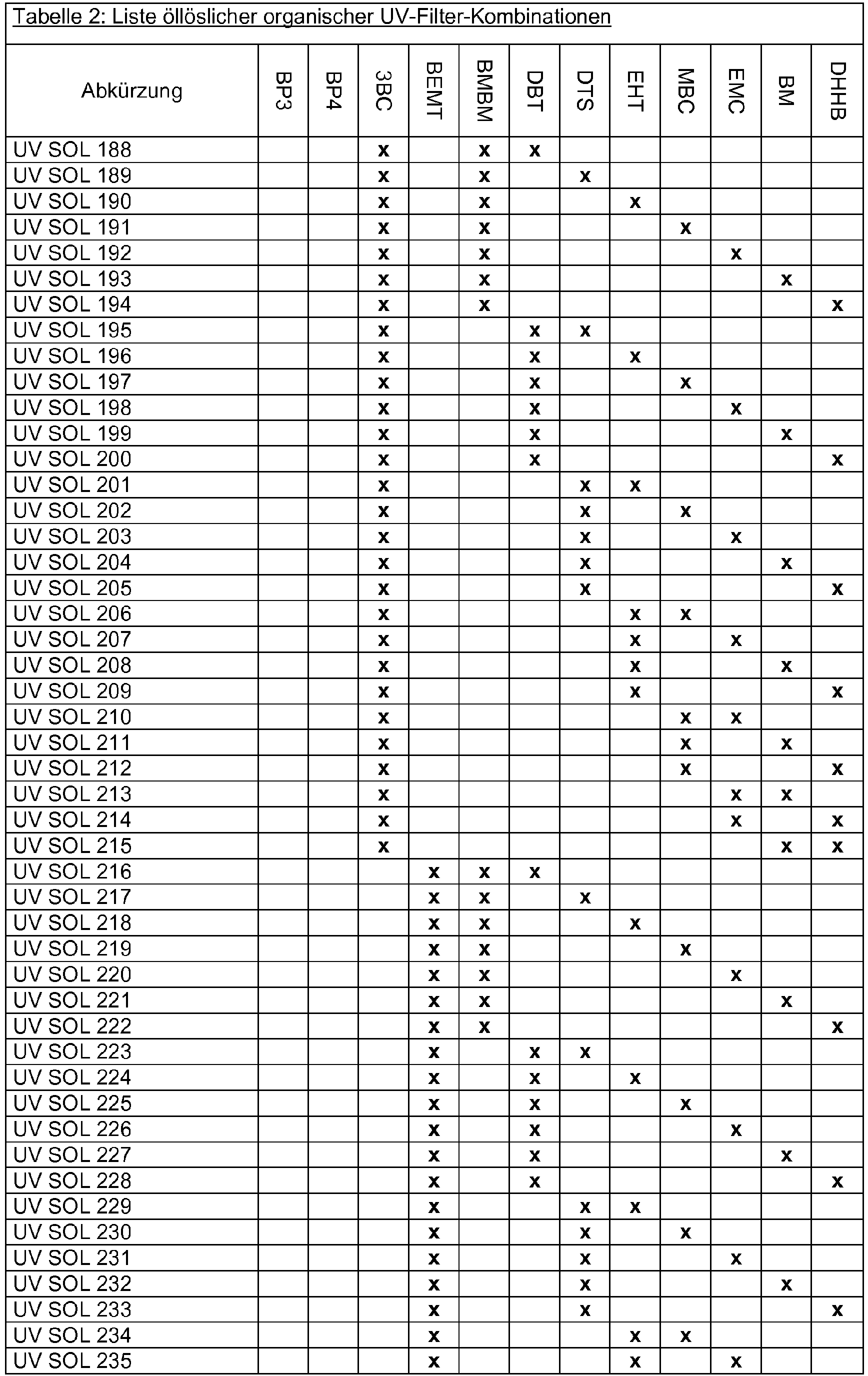

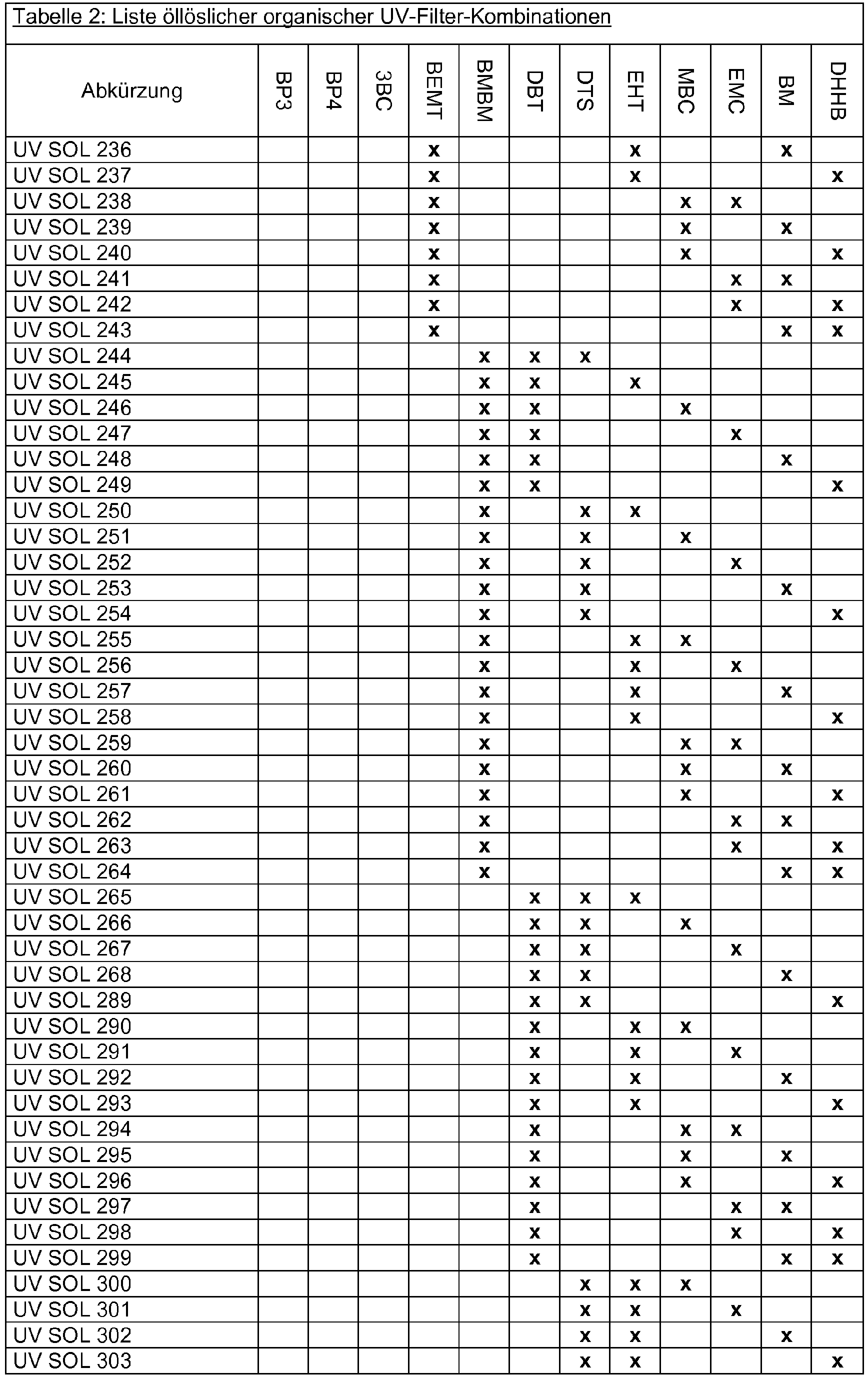

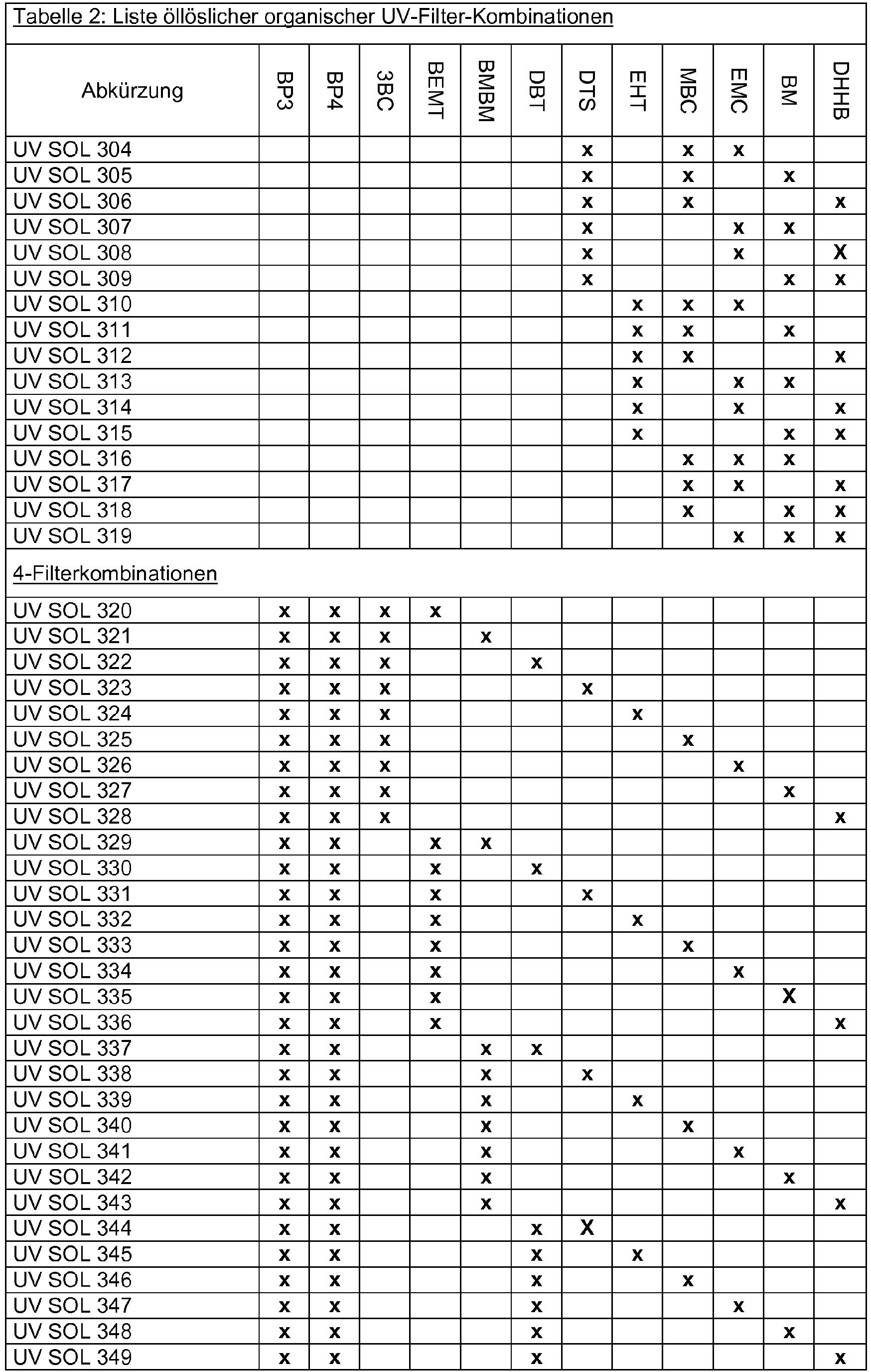

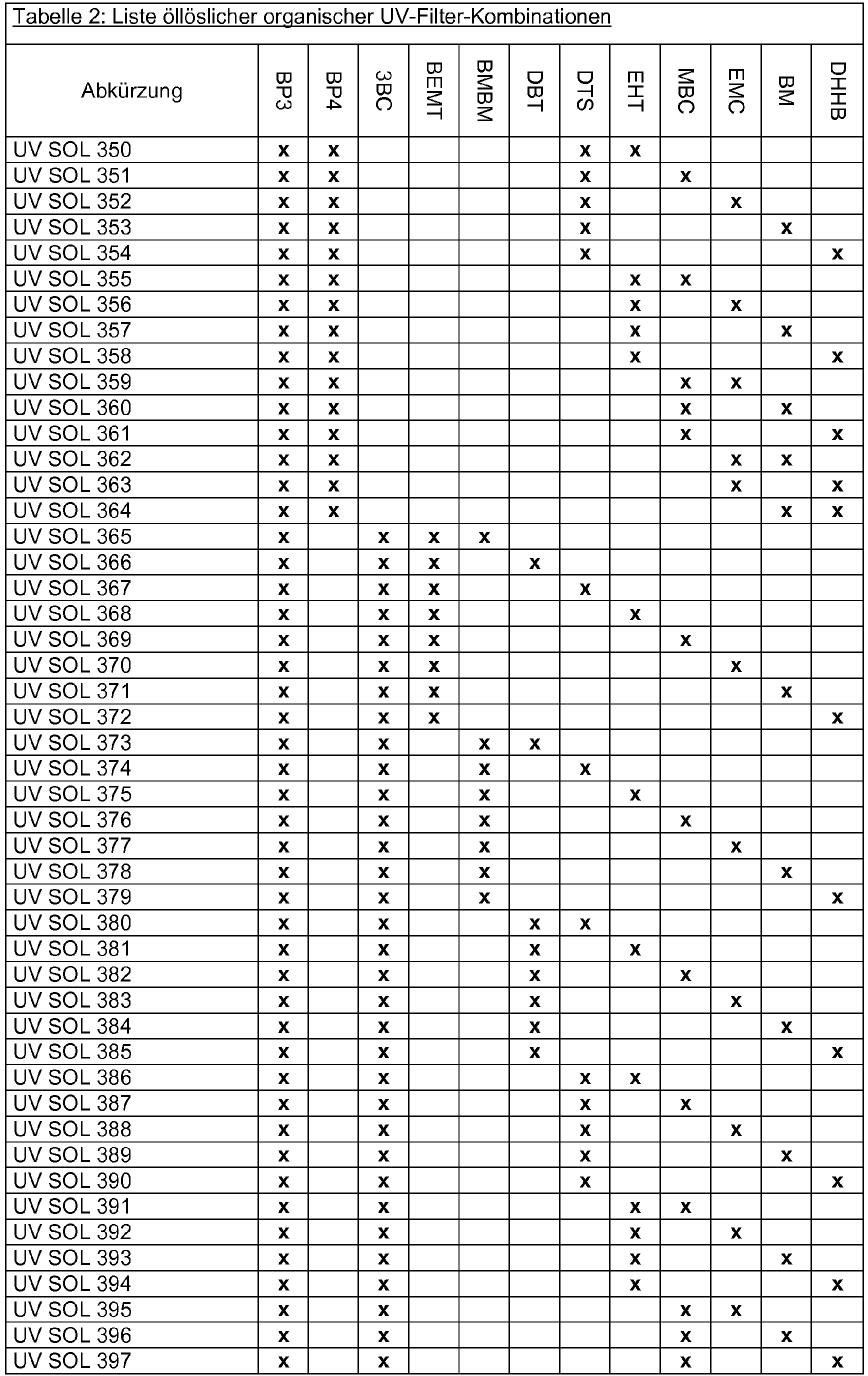

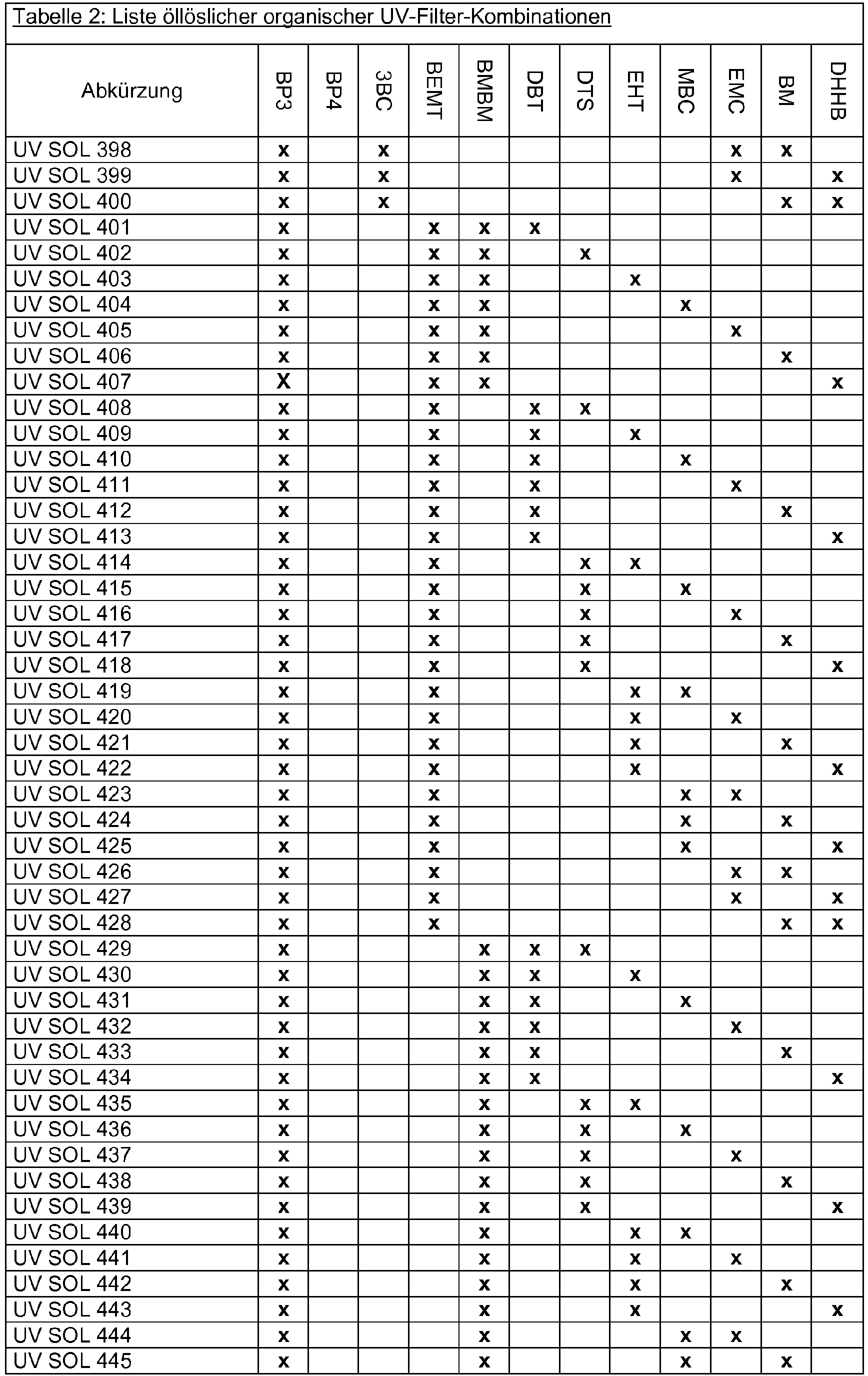

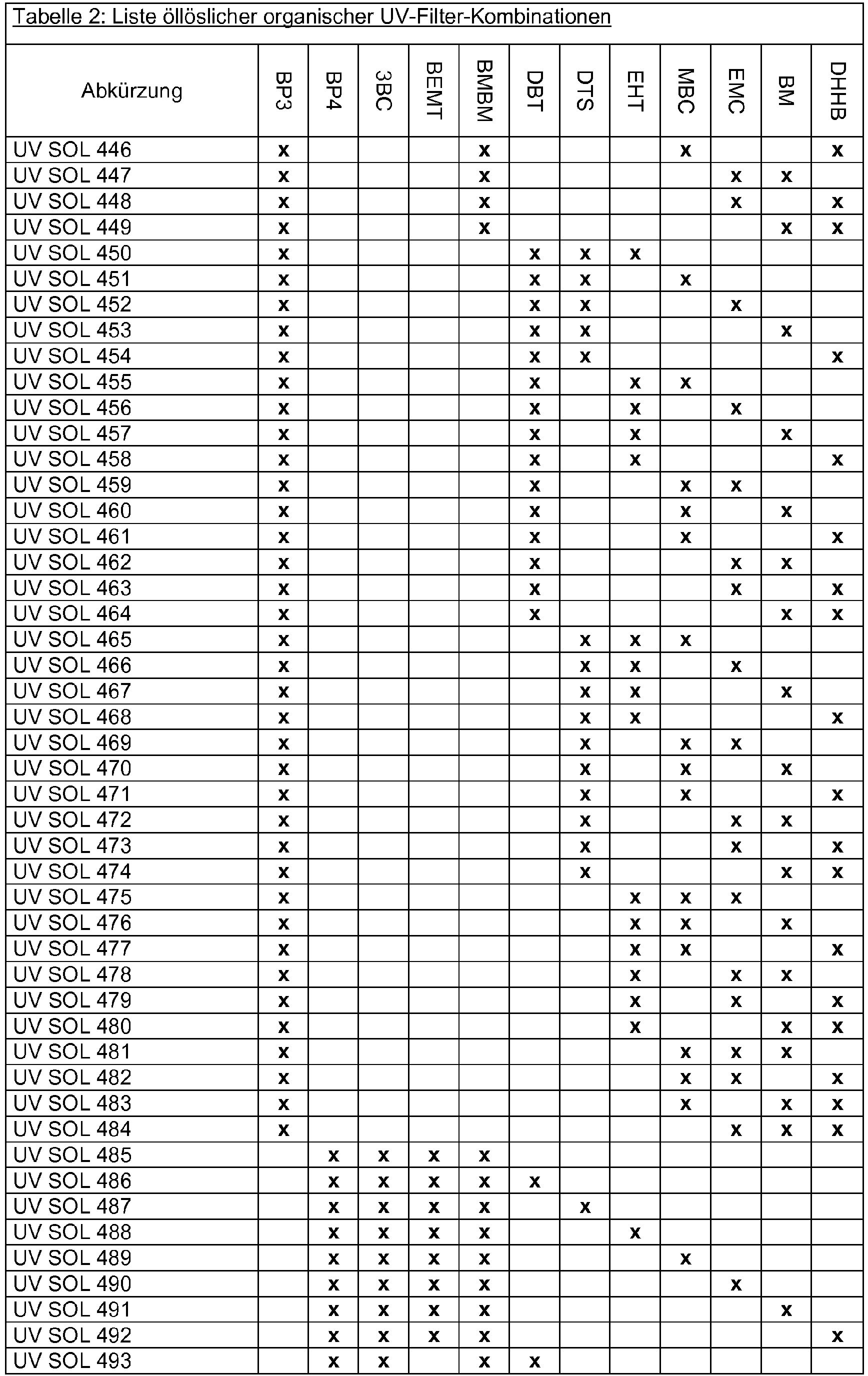

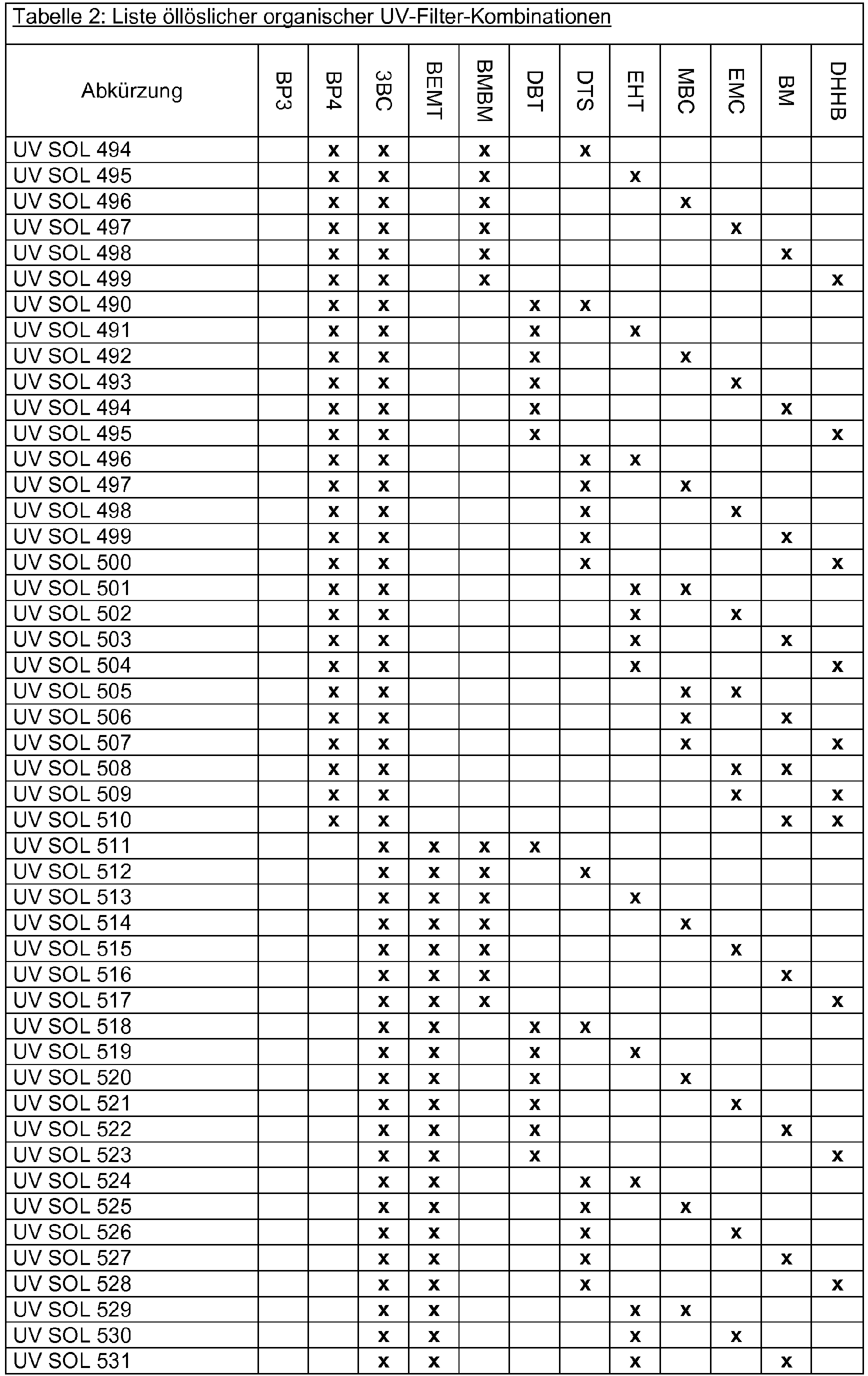

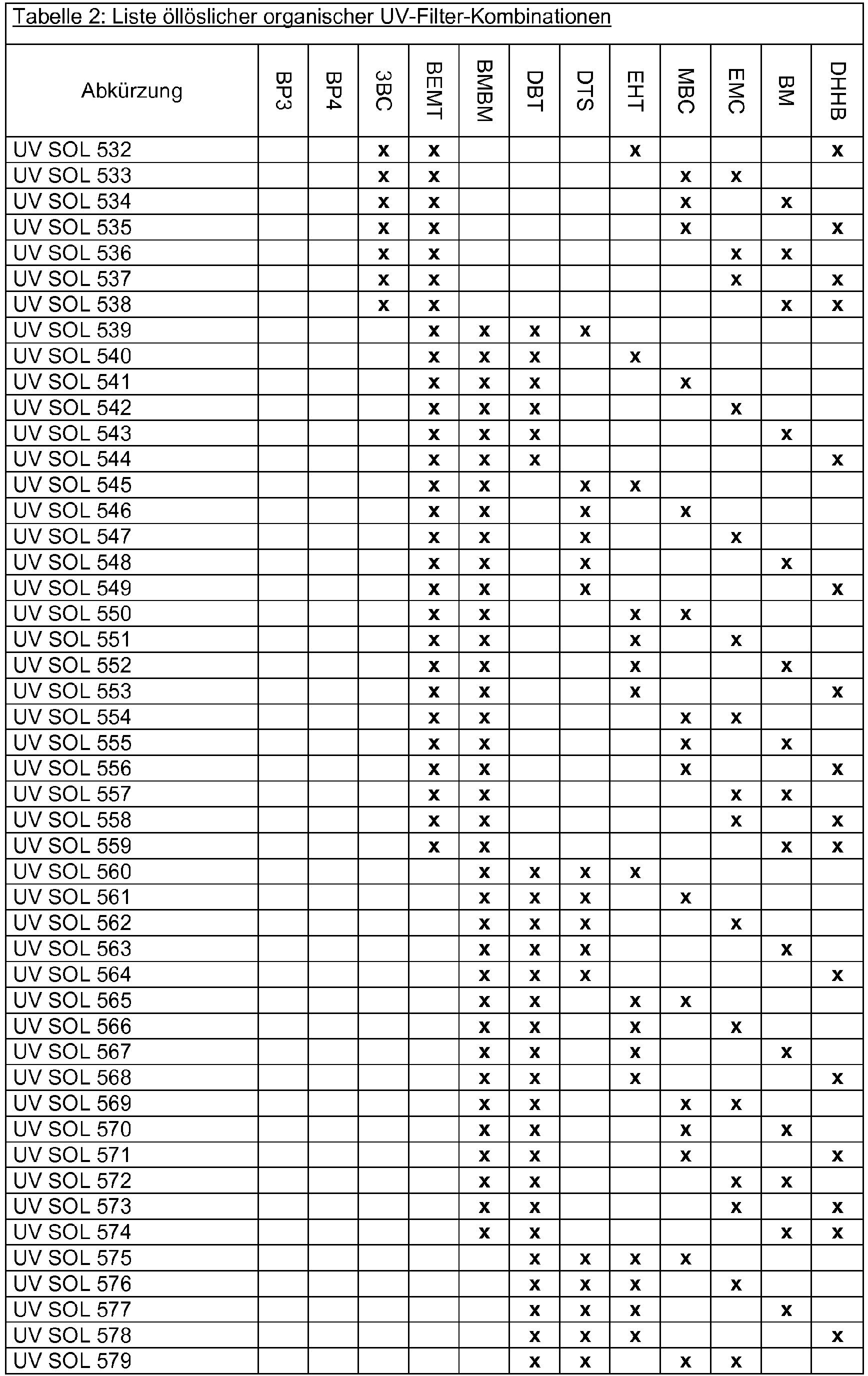

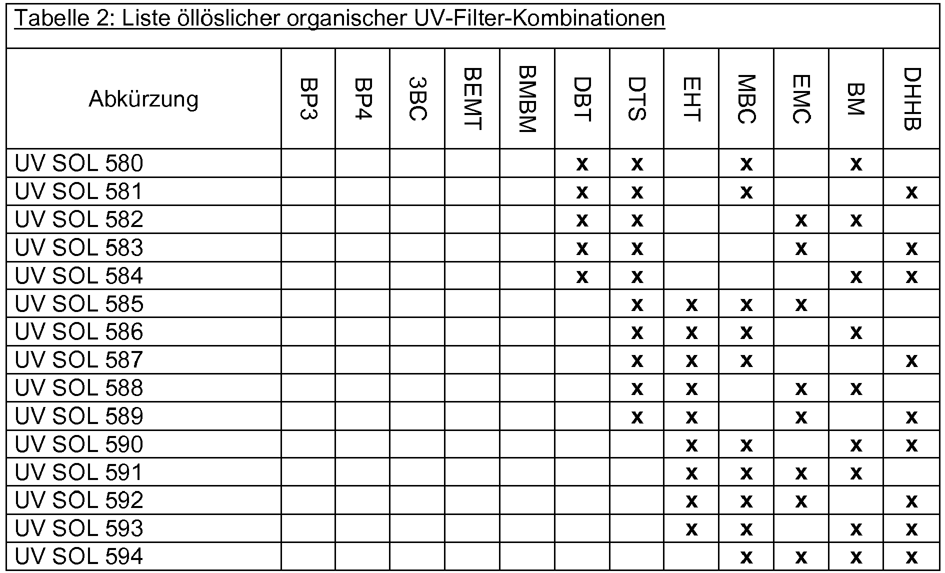

| Abkürzungen | | |
|---|---|---|
| BP3 | Benzophenone 3 | 131-57-7 |
| BP4 | Benzophenone-4 | 4065-45-6 |
| 3BC | 3-Benzydilene Camphor | 15087-24-8 |
| BEMT | Bis-Ethylhexyloxyphenol Methoxyphenyl Triazine | 103597-45-1 |
| BMBM | Butyl Methoxydibenzoylmethane | 70356-09-1 |
| DBT | Diethylhexyl Butamido Triazone | 154702-15-5 |
| DTS | Drometrizole Trisiloxane | 155633-54-8 |
| EHT | Ethylhexyl Triazone | 88122-99-0 |
| MBC | Methylbenzylidencampher | |
| EMC | Ethylhexyl Methoxycinnamate | 36861-47-9 |
| DHHB | Diethylamino Hydroxy Benzoyl Hexyl Benzoate | |

Vorteilhaft lassen sich hohe Mengen an UV-Filter einarbeiten, so dass die erfindungsgemässen Mittel ölige Konzentrate aus UV-Filtern darstellen, welche später mit gewünschten Mengen an Wasser in Sonnenschutz-Endformulierungen überführt werden können. Dabei ist die absolute Menge an UV-Filtern abhängig von der gewählten Art des UV-Filters, der gewünschten Verdünnung der Sonnenschutz-Endformulierung sowie dem gewünschten Sonnenschutzfaktor.

Obgleich das erfindungsgemässe Mittel höhere Mengen an Wasser verträgt, ist im Sinne der Erfindung ein öliges Konzentrat gewünscht, so dass die Wassermengen vorzugsweise so niedrig wie möglich sind.

Im Sinne der Erfindung ist es daher bevorzugt, dass die erfindungsgemässen Mittel kein oder nur geringe Mengen an Wasser unter 10 Gew.-%, vorzugsweise 0 bis 5 Gew.-% haben.

Mit den erfindungsgemässen Mitteln ist es nun möglich, über Menge und Auswahl der eingearbeiteten UV-Filter Sonnenschutz-Endformulierungen mit hohen SPF-Werten, vorzugsweise bis 50+ zu erzielen.

Für die Ermittlung der SPF - Werte können die Sonnenschutzsimulatoren beispielsweise der BASF Personal Care and Nutrition GmbH herangezogen werden.

Schliesslich können die erfindungsgemässen Mittel ad 100 Gew.-% Wasser und/oder Hilfsstoffe enthalten.

Hilfsstoffe können beispielsweise pH-Stellmittel sein. Beispielsweise Triethanolamin, Monoethanolamin oder Tetrahydroxypropyl Ethylendiamine. Bevorzugt ist Triethanolamin.

Erfindungsgemäss geeignet sind Mittel, die
(a) 20 bis 35 Gew.-% Ölkörper,
(b) 7 bis 17 Gew.-% anionische Tenside,
(c) 3 bis 18 Gew-% Co-Tenside,
(d) 35 bis 65 Gew.-% UV-Filter und
(e) 0,01 bis 5 Gew.-% Wasser sowie ggf. 0,5 bis 5 Gew.-% Hilfsstoffe enthalten, unter der Bedingung, dass sich alle Bestandteile auf 100 Gew.-% addieren.

Besonders bevorzugt sind Mittel, die
(a) 20 bis 35 Gew.-% eines Ölkörpers, ausgewählt aus linearen und verzweigten Fettalkoholcarbonaten, insbesondere Dicaprylyl Carbonate und/oder Ester von C₂-C₁₂-Dicarbonsäuren
(b) 7 bis 17 Gew.-% anionische Tenside, ausgewählt aus Alkylpolyalkylenglykolethercitraten, insbesondere die Polyethylenglykolether des Laurylalkohols;
(c) 3 bis 18 Gew-% Co-Tenside, ausgewählt aus nichtionische Tenside, vorzugsweise Polyol- und/oder Polyglycerinester
(d) 35 bis 65 Gew.-% UV-Filter, ausgewählt aus
   - (d₉ₐ): Ethylhexyl Methoxycinnamate,
   - (d₁₁ₐ): Bis-Ethylhexyloxyphenol Methoxyphenyl Triazine,
   - (d₁₃ₐ): Ethylhexyl Triazone und
   - (d₃ₐ): Diethylamino Hydroxy Benzoyl Hexyl Benzoate; und
(e) 0,01 bis 5 Gew.-% Wasser sowie ggf. 0,5 bis 5 Gew.-% Hilfsstoffe enthalten; wobei
die Komponenten (d₉ₐ), (d₁₁ₐ), (d₁₃ₐ) und (d₃ₐ) als Einzelverbindung oder 2-er, 3-er oder 4-er - Mischung in dem Mittel vorliegen können.

Die erfindungsgemässen Mittel können im Labormassstab durch einfaches, manuelles Rühren bei Raumtemperatur oder, falls ein bei Raumtemperatur fester Stoff enthalten ist, bei erhöhten Temperaturen, hergestellt werden. Vorzugsweise wird der Ölkörper (a) vorgelegt und die UV-Filter (d) werden untergerührt, vorzugsweise bei erhöhten Temperaturen und insbesondere bei 85 bis 95°C. Man erhält klare Mischungen, die vorzugsweise auf Raumtemperatur abgekühlt werden, bevor die anionischen Tenside (b) sowie Co-Tenside (c) und ggf. Hilfsstoffe eingerührt werden. Zusätzliches Wasser, welches nicht in Form von wässrigen Formulierungen der Tenside und/oder Hilfsstoffe eingebracht werden, kann im letzten Verfahrensschritt zuzugeben werden, ist aber im Sinne der Erfindung nicht erwünscht.

In einer weiteren bevorzugten Herstellungsweise wird der Ölkörper (a) vorgelegt und die UV-Filter (d) werden untergerührt, vorzugsweise bei erhöhten Temperaturen und insbesondere bei 85 bis 95°C. Danach werden die anionischen Tenside (b) sowie die Co-Tenside (c) eingerührt. Danach wird abgekühlt. Anschließend werden noch ggf. Hilfsstoffe, wie z.B. pH-Stellmittel oder zusätzliches Alkylpolyglucosid hinzugegeben. Man erhält hierbei eine klare Mischung.

Die klaren Konzentrate mit einem hohen Gehalt an UV-Filter(n), zeichnen sich zudem durch eine sehr hohe Lagerstabilität von mehreren Wochen aus.

Ein weiterer Gegenstand der vorliegenden Erfindung bezieht sich auf ein Verfahren zur Herstellung von Sonnenschutz-Endformulierungen. Das Verfahren ist dadurch gekennzeichnet, dass die Mittel nach Anspruch 1 mit Wasser sowie ggf. weiteren UV-Filtern und ggf. üblichen weiteren Hilfsstoffen bei Temperaturen im Bereich von 5 bis 30°C verdünnt werden.

Vorteilhafterweise wird das Verdünnen in einem sog. Kaltprozess, also bei Raumtemperatur, vorzugsweise im Bereich von 20 bis 25°C, durchgeführt, wobei die erfindungsgemässen Mittel mit Wasser sowie ggf. üblichen weiteren Hilfsstoffen in die Sonnenschutz-Endformulierungen überführt werden.

Als weitere UV-Filter können die bereits erwähnten UV-Filter (d₁) - (d₂₀) den erfindungsgemässen Mitteln hinzugefügt werden. Beispielhafte Substanzen sind in der folgenden Tabelle 3 erwähnt:

| Tabelle 3: Geeignete UV Filter und Hilfsmittel die zusätzlich verwendet werden können | | | | |
|---|---|---|---|---|
| No. | Chemische Bezeichnung | | | CAS No. |
| (2-Hydroxy-4-methoxyphenyl)(4-methylphenyl)methanone | | | | 1641-17-4 |
| Alpha-(2-oxoborn-3-ylidene)toluene-4-sulphonic acid and its salts (Mexoryl SL) | | | | 56039-58-8 |
| Methyl N,N,N-trimethyl-4-[(4,7,7-trimethyl-3-oxobicyclo[2,2,1]hept-2-ylidene)methyl]anilinium sulphate (Mexoryl SO) | | | | 52793-97-2 |
| Menthyl-o-aminobenzoate | | | | 134-09-8 |
| 2-phenyl-1H-benzimidazole-5-sulphonic acid; phenylbenzimidazolsulfonic acid | | | | 27503-81-7 |
| 2-Propenamide, N-[[4-[(4,7,7-trimethyl-3-oxobicyclo[2.2.1]hept-2-ylidene)methyl]phenyl]methyl]-, homopolymer | | | | 147897-12-9 |
| 3, 3'-(1,4-phenylenedimethylene)bis[7,7-dimethyl-2-oxo-bicyclo[2.2.1]heptane-1 methanesulfonic acid] (Cibafast H) | | | | 90457-82-2 |
| 1H-Benzimidazole-4,6-disulfonic acid, 2,2'-(1,4-phenylene)bis-, disodium salt | | | | 180898-37-7 |
| Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane (Mexoryl XL) | | | | 155633-54-8 |
| 1-Dodecanaminium, N-[3-[[4-(dimethylamino)benzoyl]amino]propyl]N,N-dimethyl-, salt with 4-methylbenzenesulfonic acid (1:1) (Escalol HP610) | | | | 156679-41-3 |
| 1-Propanaminium, N,N,N-trimethyl-3-[(1-oxo-3-phenyl-2-propenyl)amino]-, chloride | | | | 177190-98-6 |
| 1H-Benzimidazole-4,6-disulfonic acid, 2,2'-(1,4-phenylene)bis- | | | | 170864-82-1 |
| 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | | | | 340964-15-0 |
| 2-Propenoic acid, 3-(1H-imidazol-4-yl)- | | | | 104-98-3 |
| 1,2,3-Propanetriol, 1-(4-aminobenzoate) (Glyceryl PABA) | | | | 136-44-7 |
| Benzene acetic acid, 3,4-dimethoxy-a-oxo- | | | | 4732-70-1 |
| 2-Propenoic acid, 2-cyano-3,3-diphenyl-, ethyl ester | | | | 5232-99-5 |
| Anthralinic acid, p-menth-3-yl ester | | | | 134-09-8 |
| 2,2'-bis(1,4-phenylene)-1H-benzimidazole-4,6-disulphonic acid mono sodium salt or Disodium phenyl dibenzimidazole tetrasulfonate (Neo Heliopan AP) | | | | 349580-12-7 |
| Benzenesulfonic acid, 5-benzoyl-4-hydroxy-2-methoxy-, sodium salt | | | | 6628-37-1 |
| Benzoic acid, 4,4'-[[6-[[3-[1,3,3,3-tetramethyl-1-[(trimethylsilyl)oxy]-1-disiloxanyl]propyl]amino]-1,3,5-triazine-2,4-diyl]diimino]bis-, dibutyl ester | | 207562-42-3 | 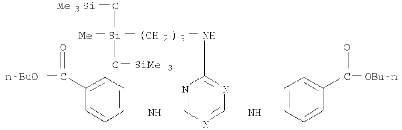 | |
| Phenol, 2-(2H-benzotriazol-2-yl)-6-[[(2-ethylhexyl)oxy]methyl]-4-methyl- | | 131411 8-38-1 | 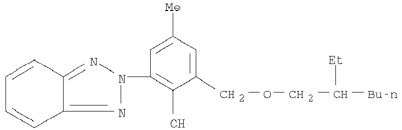 | |
| 2-Propenoic acid, 3-(4-ethoxyphenyl)-, 2-methylphenyl ester | | 433305-97-6 | 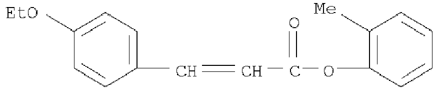 | |
| 2-Propenoic acid, 3-(4-methoxyphenyl)-, 2-methylphenyl ester | | 431067-87-7 | 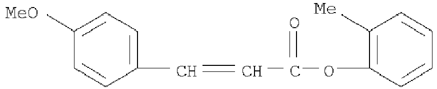 | |

| No. | Chemische Bezeichnung | | | CAS No. |
|---|---|---|---|---|
| Propanedioic acid, 2-[(4-methoxyphenyl)methylene]-, 1,3-bis(2-methylbutyl) ester | | 125730 7-15-5 | 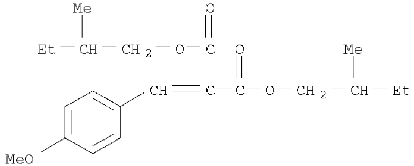 | |
| Propanedioic acid, 2-[(4-methoxyphenyl)methylene]-, 1,3-bis(2-ethylhexyl) ester | | 189183-15-1 | 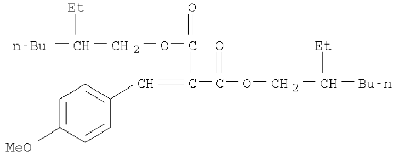 | |
| Polymere Benzotriazol UV filter wie z.B. in der US 20110195036 , WO 2011097555; beschrieben, d.h. Sonnenschutzformulierungen, enthaltend ein Polymer hergestellt aus einem dimeren Diol (C36H72O), Ditrimethylolpropan, Dimethyladipate, Mehyladipate, und Methyl-3-(2H-benzotriazol-2-yl)-5-(1,1-dimethylethyl)-4-hydroxybenzolpropanoat | | | | |
| 2-Ethoxy-2'-ethyl-oxanilid | | CAS No 23949-66-8 | 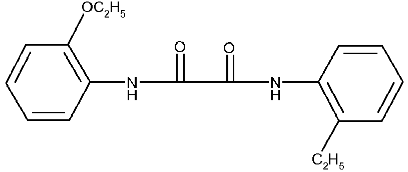 | |
| Uvinul S-Pack | | CAS 852282-89-4 | | |
| N-(2,6-Diisopropylphenyl)-6-[4-(1,1,3,3-tetramethylbutyl)phenoxy]-1H-benzo[d,e]isoquinoline-1,3(2H)-dion | | | | |
| 1,4-dihydropyridin Derivate und ionische 1,4-dihydropyridin | | | 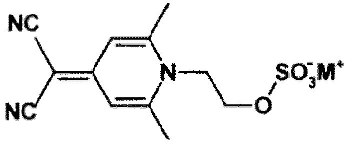 | |
| | | | worin M ein Gegenion ausgewählt aus Na⁺,K⁺, Li⁺, NH₄⁺, und 0.5 Äquivalenten Mg²⁺ und Ca²⁺, vorzugsweise Na⁺ bedeutet | |

Jeder der in der obigen Tabelle aufgezählten UV-Filter kann als zusätzliche Filter in der erfindungsgemässen Zusammensetzung verwendet werden. Es können ein, zwei, drei, vier, fünf oder sechs weitere UV-Filter eingesetzt werden.

Besonders feinteilige und hautfreundliche O/W-Sonnenschutz-Endformulierungen werden nach dem erfindungsgemässen Verfahren erhalten, wenn die erfindungsgemässen hochkonzentrierten Mittel in Mengen von 6 bis 50 Gew.%, vorzugsweise > 10 bis 50 Gew.%, und insbesondere von 15 bis 50 Gew.%, bezogen auf O/W-Sonnenschutz-Endformulierung, mit Wasser sowie ggf. Hilfsmittel homogenisiert werden.

Die Homogenisierung erfolgt vorzugsweise unter moderater Mechanik, also durch einfaches Rühren. Besondere Scherkräfte sind möglich, aber nicht notwendig.

Es hat sich als vorteilhaft erwiesen, wenn während der Verdünnung mit Wasser bereits Verdicker als weitere Hilfsmittel zugegen sind. Als Verdicker eignen sich anionische, zwitterionische, amphotere und nichtionische Copolymere wie beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, Acrylamidopropyltrimethylammoniumchlorid/Acrylat-Copolymere, Octylacrylamid/Methylmethacrylat/tert.-Butylaminoethylmethacrylat/2-Hydroxypropylmethacrylat-Copolymere, Vinylpyrrolidon/Vinylacetat-Copolymere, Vinylpyrrolidon/Dimethylaminoethylmethacrylat/Vinylcaprolactam-Terpolymere sowie gegebenenfalls Polysaccharide, insbesondere Xanthan-Gum, Guar und Guarderivate, Agar-Agar, Alginate und Tylosen, Cellulose und Cellulosederivate wie Carboxymethylcellulose, Carboxymethylcellulose und Hydroxycellulose und ausserdem Silicone. Vorzugsweise werden Verdicker aus der Gruppe der Polyacrylate bzw. vernetzten Polyacrylaten wie Rheocare TTA®, Cosmedia® SP,, Rheocare® C Plus, Tinovis® ADE, Tinovis® GTC, zugesetzt und zwar vorzugsweise in Mengen von 0,5 bis 5, insbesondere von 1, bis 4 Gew.%-berechnet als Aktivsubstanz und bezogen auf Sonnenschutz-Endformulierung. Weiterhin bevorzugt sind Verdicker aus der Gruppe der Polysaccharide wie Keltrol® T oder Rheocare® XG.

Die Verdicker können dem konzentrierten Mittel zugegeben werden, bevor die Verdünnung mit Wasser erfolgt oder im Wasser enthalten sein, mit dem die Verdünnung des konzentrierten Mittels erfolgt.

Nach einer bevorzugten Verfahrensvariante wird das konzentrierte Mittel mit dem Verdicker vermischt und hierzu Wasser zur Verdünnung gegeben sowie ggf. die weiteren Formulierungsbestandteile eingerührt.

Nach einer anderen bevorzugten Verfahrensvariante werden das Wasser, der Verdicker sowie ggf. die anderen Hilfsstoffe miteinander verrührt und hierzu das konzentrierte Mittel gegeben.

Die nach dem erfindungsgemässen Verfahren hergestellten Sonnenschutz-Endformulierungen sind oft besonders feinteilige O/W-Emulsion mit einer mittleren Teilchengrösse < 10 µm, vorzugsweise < 5 µm.

Ein weiterer Gegenstand der vorliegenden Erfindung betrifft die Verwendung der konzentrierten Mittel nach Anspruch 1 zur Herstellung von kosmetischen Formulierungen mit einem sehr hohem Sonnenschutzfaktor, vorzugsweise mit einem SPF bis zu 50+.

Falls gewünscht, können die Sonnenschutz-Endformulierungen weitere Hilfsstoffe wie Feuchthalte/Hautbefeuchtungsmittel, Viskositätsregulatoren, Öle, Fette und Wachse, Tenside, Perlglanzwachse, Überfettungsmittel, Stabilisatoren, kationische, zwitterionische oder amphotere Polymere, weitere UV-Filter, biogene Wirkstoffe, Filmbildner, Quellmittel, Hydrotrope, Konservierungsmittel, Solubilisatoren, Parfümöle, Farbstoffe, Insektenrepellent-Wirkstoffe etc. enthalten, die nachstehend beispielhaft aufgeführt sind.

Feuchthaltemittel dienen zur weiteren Optimierung der sensorischen Eigenschaften der Zusammensetzung sowie zur Feuchtigkeitsregulierung der Haut. Die Feuchthaltemittel können in einer Menge von 0 - 5 Gew.-% enthalten sein. Geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäss bevorzugt geeignet als Feuchthaltemittel sind Glycerin, Diglycerin, Triglycerin und Butylenglycol. Als Insekten-Repellentien kommen beispielsweise N,N-Diethyl-m-toluamid, 1,2-Pentandiol oder 3-(N-n-Butyl-N-acetyl-amino)-propionic acid-ethyl ester), welches unter der Bezeichnung Insect Repellent 3535 von der Merck KGaA vertrieben wird, sowie Butylacetylaminopropionate in Frage. Sie werden in den erfindungsgemässen Zusammensetzungen üblicherweise in einer Menge von 0 - 6 Gew.-%, bezogen auf Mittel, eingesetzt.

Die Viskosität der erfindungsgemässen Mittel kann durch Zugabe von Viskositätsregulatoren erreicht werden. Als Viskositätsregulatoren kommen u.a. Konsistenzgeber, wie z. B. Fettalkohole oder Hydroxyfettalkohole mit 12 bis 22 und vorzugsweise 16 bis 18 Kohlenstoffatomen sowie Partialglyceride, Fettsäuren mit 12 bis 22 Kohlenstoffatomen oder 12-Hydroxyfettsäuren in Betracht. Geeignet ist auch eine Kombination dieser Stoffe mit Alkyloligoglucosiden und/oder Fettsäure-N-methylglucamiden gleicher Kettenlänge, da derartige Kombinationen besonders stabile und homogene Emulsionen liefern. Zu den Viskositätsregulatoren zählen auch Verdickungsmittel wie beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), unvernetzte und mit Polyolen vernetzte Polyacrylsäuren, Polyacrylamide, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Auch Tenside, wie beispielsweise ethoxylierte Fettsäureglyceride, Ester von Fettsäuren mit Polyolen, wie beispielsweise Pentaerythrit oder Trimethylolpropan, Fettalkoholethoxylate mit eingeengter Homologenverteilung, Alkyloligoglucoside sowie Elektrolyte, wie z. B. Kochsalz und Ammoniumchlorid können zur Viskositätsregulierung eingesetzt werden.

Unter Fetten und Wachsen werden im Sinne der Erfindung alle Lipide mit fett- oder wachsartiger Konsistenz verstanden, die einen Schmelzpunkt oberhalb von 20°C aufweisen. Hierzu gehören beispielsweise die klassischen Triacylglycerine, also die Dreifachester von Fettsäuren mit Glycerin, die pflanzlicher oder tierischer Herkunft sein können. Hierbei kann es sich auch um Mischester, also um Dreifachester aus Glycerin mit verschiedenen Fettsäuren, oder aber um ein Gemisch verschiedener Glyceride handeln. Hierzu gehören auch Gemische aus Mono- Di- und Triglyceriden. Erfindungsgemäss besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnussöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett. Besonders geeignet sind oxidationsstabile pflanzliche Glyceride, die unter der Bezeichnung Cegesoft® oder Novata® angeboten werden.

Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Neben den Fetten kommen als Zusatzstoffe auch fettähnliche Substanzen, wie Lecithine und Phospholipide in Frage. Lecithine sind Glycero-Phospholipide, die sich aus Fettsäuren, Glycerin, Phosphorsäure und Cholin durch Veresterung bilden, und häufig auch als Phosphatidylcholine (PC) bezeichnet werden. Als Beispiel für natürliche Lecithine seien die Kephaline genannt, die auch als Phosphatidsäuren bezeichnet werden und Derivate der 1,2-Diacyl-*sn-*glycerin-3-phosphorsäuren darstellen. Dem gegenüber versteht man unter Phospholipiden gewöhnlich Mono- und vorzugsweise Diester der Phosphorsäure mit Glycerin (Glycerinphosphate). Auch Sphingosine bzw. Sphingolipide kommen als fettartige Stoffe in Frage.

Als Perlglanzwachse geeinget sind beispielsweise Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierten Carbonsäuren mit C₆-C₂₂-Fettalkoholen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen - speziell Lauron®; Distearylether; Fettsäuren wie Stearinsäure, C₁₂-C₂₂-Hydroxyfettsäuren, Behensäure, Ringöffnungsprodukte von C₁₂-C₂₂-Olefinepoxiden mit C₁₂-C₂₂-Fettalkoholen und/oder Polyolen mit 2 bis 15 Kohlenstoffatomen und 2 bis 10 Hydroxylgruppen sowie deren Mischungen.

Als Überfettungsmittel können Substanzen wie bespielsweise Lanolin und Lecithin sowie polyethoxylierte oder acylierte Lanolin- und Lecithinderivate, Polyolfettsäureester, Monoglyceride und Fettsäurealkanolamide verwendet werden, wobei letztere gleichzeitig als Schaumstabilisatoren dienen.

Als sogenannte Stabilisatoren können Metallsalze von Fettsäuren, wie z.B. Magnesium-, Aluminium- und/oder Zinkstearat bzw. -ricinoleat eingesetzt werden.

Geeignete kationische Polymere, welche die Sensorik der erfindungsgemässen Zusammensetzungen weiter optimieren und der Haut ein Gefühl der Weichheit verleihen, sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Weiterhin können Stärkederivate zur Verbesserung des Hautgefühls eingesetzt werden, z.B. Dry Flo® PC (INCI: Aluminum Starch Octenylsuccinate).

Geeignete Siliconverbindungen wurden bereits bei den Ölkörpern erwähnt. Neben Dimethylpolysiloxanen, Methylphenylpolysiloxanen und cyclischen Siliconen sind auch amino-, fettsäure-, alkohol-, polyether-, epoxy-, fluor-, glykosid- und/oder alkylmodifizierte Siliconverbindungen geeignet, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und Siliciumdioxid oder hydrierten Silicaten handelt.

Erfindungsgemäss geeignete biogene Wirkstoffe sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen. Derartige Wirkstoffe werden als Radikalfänger in Sonnenschutz-Endformulierungen eingesetzt und dienen der Regeneration der Haut.

Sogenannte Filmbildner, die zu einer weiteren Verbesserung der Sensorik der erfindungsgemässen Zubereitungen führen, sind beispielsweise Chitosan, mikrokristallines Chitosan, quaterniertes Chitosan, Kollagen, Hyaluronsäure bzw. deren Salze und ähnliche Verbindungen, sowie die bereits unter den Viskositätsregulatoren genannten Polyvinylpyrrolidone, Vinylpyrrolidon-Vinylacetat-Copolymerisate, Polymere der Acrylsäurereihe und quaternäre Cellulose-Derivate.

Zur Verbesserung des Fliessverhaltens der erfindungsgemässen Zusammensetzungen können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe.

Als Parfümöle seien genannt natürliche, pflanzliche und tierische sowie synthetische Riechstoffe oder deren Gemische. Natürliche Riechstoffe werden u.a. durch Extraktion von Blüten, Stengeln, Blättern, Früchten, Fruchtschalen, Wurzeln und Harzen von Pflanzen erhalten. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Bevorzugt werden Mischungen verschiedener Riechstoffe verwendet, die gemeinsam eine ansprechende Duftnote erzeugen.

Als Farbstoffe können die für kosmetische Zwecke geeigneten und zugelassenen Substanzen verwendet werden.

Die nachfolgenden Beispiele dienen zur Veranschaulichung der Erfindung, ohne diese auf die Beispiele zu beschränken.

### Beispiele A1 bis A7: UV-Filter enthaltende Konzentrate

### Angaben kalkuliert als Aktivsubstanz in Gew.-%

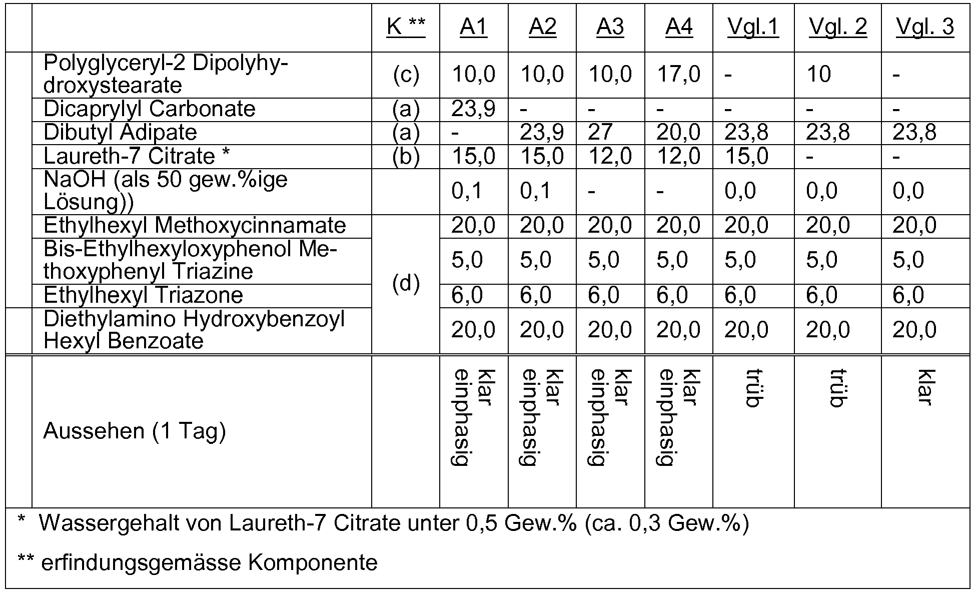

Die erfindungsgemässen Konzentrate werden hergestellt durch Vermischen der Ölkomponente (a) (Dicaprylyl Carbonate oder Dibutyl Adipate) mit den UV- Filtern (d) (Ethylhexyl Methoxycinnamate, Bis-Ethylhexyloxyphenol Methoxyphenyl Triazine, Ethylhexyl Triazone, Diethylamino Hydroxybenzoyl Hexyl Benzoate) unter Erwärmen auf 90 bis 95°C. Nach Erhalten einer klaren Lösung wird auf Raumtemperatur abgekühlt und das Aniontensid (b) (Laureth-7 Citrate) sowie das Co-Tensid (c) (Polyglyceryl-2 Dipolyhydroxystearate) untergerührt.

Es werden lagerstabile Konzentrate erhalten, die auch nach mehrwöchiger Lagerung bei Raumtemperatur keinen Bodensatz oder eine Bildung von Kristallen der UV-Filter aufweisen.

Als Vergleich werden entsprechende Konzentrate hergestellt, jedoch ohne anionisches Tensid (b) (Laureth-7 Citrate) gemäss Vgl. 2 bzw. ohne Co-Tensid (c) (Polyglyceryl-2 Dipolyhydroxystearate) gemäss Vgl. 1 bzw. ganz ohne Tenside gemäss Vgl. 3. Das Konzentrat gemäss Vgl. 1 ist trüb, das Konzentrat gemäss Vgl. 2 leicht trüb. Das Konzentrat gemäss Vgl. 3 bildet unerwünschte Kristalle beim Lagern innerhalb von 4 Wochen. Weiterhin ist das Konzentrat gemäss Vergl. 3 nicht selbstemulgierend beim Verdünnen mit Wasser.

### Beispiele A6 - A9: weitere Bespiele für Konzentrate (Angaben in Gew.-% Produkt)

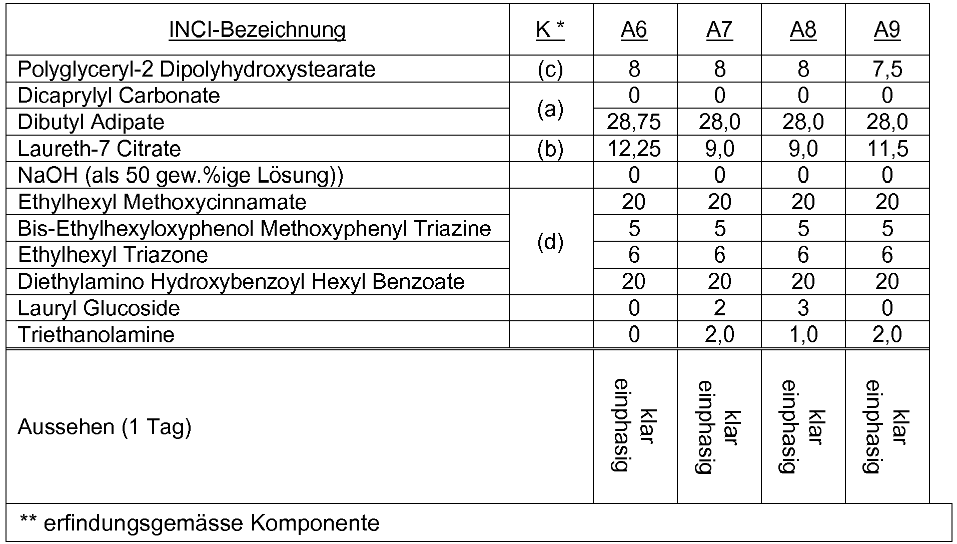

### Beispiele B1 bis B3: O/W Sonnenschutz-Endformulierungen auf Basis der UV-Filter enthaltenden Konzentrate (Angaben kalkuliert als Aktivsubstanz in Gew.-%)

| Verbindung | B1 | B2 | B3 | Vgl. B |
|---|---|---|---|---|
| Rheocare TTA® Acrylates copolymer (Verdicker) | 3,5 | | - | - |
| Cosmedia® SP Sodium Polyacrylate (Verdicker) | | 1,5 | - | - |
| Konzentrat nach Beispiel A3 | 30 | 30 | | - |
| Konzentrat nach Beispiel A4 | | | 10 | |
| Konzentrat nach Vgl. 3 | | | | 10 |
| Glycerin | - | 5,0 | | - |
| Magnesiumsulfat (fest) | - | 0,5 | | - |
| NaOH (als 50 gew.%ige Lösung)) | qs | qs | | -- |
| Konservierungsmittel | qs | qs | qs | - |
| Aqua dem. | Ad 100 | Ad 100 | Ad 100 | Ad 100 |
| Aussehen (1 Tag) | weiss | weiss | weiss | phasensep. |
| Struktur | O/W Emulsion | O/W Emulsion | O/W Emulsion | entmischt |
| Teilchendurchmesser | < 5 µm | < 5 µm | < 5 µm | - |
| SPF calc | 30,2 ratio 2,44 | 30,2 ratio 2,44 | 9,8 ratio 1,81 | |

Für das Sonnenschutzmittel B1 wird der Verdicker mit Wasser und allen anderen Bestandteilen ausser dem Konzentrat homogenisiert und vorgelegt. Hierzu wird anschliessend das Konzentrat nach Beispiel A3 zugegeben und alles mit dem Magnetrührer homogenisiert. Der pH-Wert wird auf 6,5 eingestellt.

Für das Sonnenschutzmittel B2 wird der Verdicker mit dem Konzentrat nach Beispiel A3 vermischt und vorgelegt. Anschliessend wird Wasser hinzugegeben sowie die restlichen Rezeptursubstanzen und alles mit dem Magnetrührer homogenisiert. Der pH-Wert wird auf 5,0 eingestellt.

Für das Sonnenschutzmittel B3, das keinen Verdicker enthält, werden Wasser sowie die restlichen Rezeptursubstanzen hinzugegeben und alles mit dem Magnetrührer homogenisiert. Der pH-Wert wird auf 5,0 eingestellt.

Zu Vergleichszwecken werden die Konzentrate aus Tabelle 1 Vgl.1, Vgl. 2 und Vgl. 3 in Wasser dispergiert, um die selbstemulgierenden Eigenschaften zu überprüfen. Während mit den erfindungsgemässen Konzentraten feinteilige O/W-Emulsionen mit einem Teilchendurchmesser im Bereich kleiner 5 µm gebildet werden, sind die Dispersionen mit den Vergleichskonzentraten instabil und es kommt nach einem Tag zu einer deutlichen Phasenseparation. Insbesondere Vgl. 3 bildet in der Verdünnung mit Wasser keine Emulsion.

### Beispiele C1 - C14: Weitere Rezepturbeispiele für die Sonnenschutzprodukte

| Verbindung | INCI-Bezeichnung | Beispiel | | | |
|---|---|---|---|---|---|
| | | C1 | C2 | C4 | C4 |
| Cosmedia® SP (Verdicker) | Sodium Polyacrylat | 2,0 | 2,0 | 2,0 | 2,0 |
| Konzentrat nach Beispiel 6 | | 50,0 | 30,0 | 15,0 | 8,0 |
| EDTA BD | Disodium EDTA | 0,2 | 0,2 | 0,2 | 0,2 |
| NaOH (als 30 gew.%ige Lösung) | Aqua, Sodium hydroxide | | | | |
| Phenonip (Konservierungsmittel) | Phenoxyethanol/Methylparaben/Ethylparaben/Butylparaben/Propylparaben/Isobutylparaben | 1,0 | 1,0 | 1,0 | 1,0 |
| Aqua dem. | | Ad 100 | Ad 100 | Ad 100 | Ad 100 |
| Aussehen (1 Tag) | | weiss | weiss | weiss | weiss |
| Struktur | | O/W Emul sion | O/W Emul sion | O/W Emul sion | O/W Emul sion |
| SPF berechnet | | 50,2 ratio 2,61 | 30,2 ratio 2,44 | 14,8 ratio 2,01 | 7,9 ratio 1,7 |

| Verbindung | INCI-Bezeichnung | Beispiel | | | |
|---|---|---|---|---|---|
| | | C5 | C6 | C7 | C8 |
| Tinovis ADE® (Verdicker) | Sodium Acrylates Copolymer Hydrogenated Polydecene PPG-1 Trideceth-6-Hydrogenated Polydecene PPG-1 Trideceth-6 | 2,0 | 2,5 | | |
| Tinovis GTC® (Verdicker) | Acrylates/Beheneth-25 Methacrylate Copolymer | | | | |
| Aristoflex HMB® (Verdicker) | Ammonium Acryloyldimethyltaurate/Beheneth-25 Methacrylate Crosspolymer | | | 0,8 | 0,8 |
| Konzentrat nach Beispiel 6 | | 50,0 | 8,0 | 50,0 | 6,0 |
| EDTA BD | Disodium EDTA | 0,2 | 0,2 | 0,20 | 0,20 |
| NaOH (als 30 gew.%ige Lösung) | Aqua, Sodium hydroxide | 3,3 | 0,90 | 2,70 | 0,35 |
| Phenonip (Konservierungsmittel) | Phenoxyethanol/Methylparaben/Ethylparaben/Butylparaben/Propylparaben/Iso butylparaben | 1,0 | 1,0 | 1,0 | 1,0 |
| Aqua dem. | | Ad 100 | Ad 100 | Ad 100 | Ad 100 |
| Aussehen (1 Tag) | | weis s | weis s | weis s | weis s |
| Struktur | | O/W Emul sion | O/W Emul sion | O/W Emul sion | O/W Emul sion |
| SPF berechnet | | 50,2 ratio 2,61 | 7,9 ratio 1,7 | 50,2 ratio 2,61 | 6,1 ratio 1,59 |

| Verbindung | INCI-Bezeichnung | Beispiel | | | |
|---|---|---|---|---|---|
| | | C9 | C10 | C11 | C12 |
| Rheocare XG® (Verdicker) | Xanthan Gum | 0,5 | 0,5 | | |
| Tinovis GTC® (Verdicker) | Acrylates/Beheneth-25 Methacrylate Copolymer | | | 3,0 | 3,0 |
| Rheocare C Plus (Verdicker) | Carbomer | | | | |
| Konzentrat nach Beispiel 6 | | 6,0 | 50,0 | 50,0 | 6,0 |
| EDTA BD | Disodium EDTA | 0,2 | 0,2 | 0,2 | 0,2 |
| NaOH (als 30 gew.%ige Lösung) | Aqua, Sodium hydroxide | | | 3,1 | 1,02 |
| Citric Acid (30%) | Aqua, Citric acid | | | 0,40 | |
| Phenonip Konservierungsmittel | Phenoxyethanol/Methyl-paraben/Ethylparaben/-Butylparaben/Propylparabe n/Isobutylparaben | 1,0 | 1,0 | 1,0 | 1,0 |
| Aqua dem. | | Ad 100 | Ad 100 | Ad 100 | Ad 100 |
| Aussehen (1 Tag) | | weiss | weiss | weiss | weiss |
| Struktur | | O/W Emul sion | O/W Emul sion | O/W Emul sion | O/W Emul sion |
| SPF berechnet | | 6,1 ratio 1,59 | 50,2 ratio 2,61 | 50,2 ratio 2,61 | 6,1 ratio 1,59 |

| Verbindung | | INCI-Bezeichnung | | Beispiel | |
|---|---|---|---|---|---|
| | | | | C13 | C14 |
| Rheocare XG® (Verdicker) | | Xanthan Gum | | | |
| Tinovis GTC® (Verdicker) | | Acrylates/Beheneth-25 Methacrylate Copolymer | | | |
| Rheocare C Plus (Verdicker) | | Carbomer | | 1,50 | 0,80 |
| Konzentrat nach Beispiel 6 | | | | 50,0 | 6,0 |
| EDTA BD | | Disodium EDTA | | 0,2 | 0,2 |
| NaOH (als 30 gew.%ige Lösung) | | Aqua, Sodium hydroxide | | 5,6 | 1,50 |
| Citric Acid (30%) | | Aqua, Citric acid | | 2,4 | |
| Phenonip (Konservierungsmittel) | | Phenoxyethanol/Methylparaben/Ethylparaben/Butylparaben/Propylparaben/I sobutylparaben | | 1,0 | 1,0 |
| Aqua dem. | | | | Ad 100 | Ad 100 |
| Aussehen (1 Tag) | | | | weiss | weiss |
| Struktur | | | | O/W Emul sion | O/W Emul sion |
| SPF berechnet | | | | 50,2 ratio 2,61 | 6,1 ratio 1,59 |

| Verbindung | INCI | Beispiel | | | |
|---|---|---|---|---|---|
| | | C15 | C16 | C17 | C18 |
| EUMULGIN PRISMA | Disodim Cetearyl Sulfosuccinate | | | | |
| EUMULGIN SG | Sodium Stearoyl Glutamate | | | | |
| AMPHISOL K | Potasium Cetyl Phosphate | | | | |
| CETIOL CC | Dicaprylyl Carbonate | 5,00 | | | |
| Konzentrat nach Beispiel 6 | | 50,00 | 50,00 | 50,00 | 50,00 |
| NaOH 30% | Aqua, Sodium hydroxide | 2,62 | 3,00 | 3,00 | 3,10 |
| Trisaminosolution (30%) | Aqua, Thromethamine | | | | |
| EDTA BD | Disodium EDTA | 0,20 | 0,20 | 0,20 | 0,20 |
| TINOVIS ADE | Sodium Acrylates Copolymer/Hydrogenated Polydecene/PPG-1 Trideceth-6 | 2,60 | 2,50 | 2,60 | 2,50 |
| RHEOCARE XG | Xanthan Gum | | | | |
| DC246 | Cyclopentasiloxane | | | | |
| DRY FLO PC | Aluminum Starch Octenylsuccinate | | | | |
| EUSOLEX 232 | Phenylbenzimidazole Sulfonic Acid | | | | |
| TRIS AMINO ULTRA PURE | Thromethamine | | | | |
| NaOH 30% | Aqua, Sodium hydroxide | | | | |
| Citric acid 30% | Aqua, Citric acid | | | | |
| TINOSORB®M | Methylene Bis-Benzotriazolyl Tetramethylbutylphenol | | 5,00 | | |
| TINOSORB®A2B | Tris-Biphenyl Triazine | | | 5,00 | |
| TINOSORB®S-AQUA | Bis-Ethylhexyloxyphenol Methoxyphenyl Triazine Polymethyl Methacrylate | | | | 5,00 |
| PHENONIP | Phenoxyethanol/Methylparaben/Ethylparaben/Butyl- | 1,00 | 1,00 | 1,00 | 1,00 |

| Verbindung | INCI | Beispiel | | | |
|---|---|---|---|---|---|
| | | C15 | C16 | C17 | C18 |
| | paraben/Propylparaben/Isobutylparaben | | | | |
| Aqua dem. | | Ad 100 | Ad 100 | Ad 100 | Ad 100 |
| Aussehen (1 Tag) | | weiss | weiss | weiss | weiss |
| Struktur | | O/W Emulsion | O/W Emulsion | O/W Emulsion | O/W Emulsion |
| SPF berechnet | | 50,2 ratio 2,61 | 60,2 ratio 2,59 | 61,9 ratio 2,59 | 57,7 ratio 2,75 |

| Verbindung | INCI | Beispiel | | | | |
|---|---|---|---|---|---|---|
| | | C19 | C20 | C21 | C22 | C23 |
| EUMULGIN PRISMA | Disodim Cetearyl Sulfosuccinate | | | 1,50 | | |
| EUMULGIN SG | Sodium Stearoyl Glutamate | | | | 1,50 | |
| AMPHISOL K | Potasium cetyl Phosphate | | | | | 1,50 |
| CETIOL CC | Dicaprylyl Carbonate | | 5,00 | 5,00 | 5,00 | 5,00 |
| Konzentrat nach Beispiel 6 | | 50,00 | 50,00 | 50,00 | 50,00 | 50,00 |
| NaOH 30% | Aqua, Sodium hydroxide | | 2,90 | 2,30 | 2,25 | 2,50 |
| Trisaminosolution (30%) | Aqua,Tromethamine | 6,50 | | | | |
| EDTA BD | Disodium EDTA | 0,20 | 0,20 | 0,20 | 0,20 | 0,20 |
| TINOVIS ADE | Sodium Acrylates Copolymer/Hydrogenated Polydecene/PPG-1 Trideceth-6 | 2,50 | 1,50 | 1,50 | 0,90 | 0,65 |
| RHEOCARE XG | Xanthan Gum | | | | | |
| DC246 | Cyclopentasiloxane | | 2,00 | 2,00 | 2,00 | 2,00 |
| DRY FLO PC | Aluminum Starch Octenylsuccinate | | 2,00 | 2,00 | 2,00 | 2,00 |
| EUSOLEX 232 | Phenylbenzimidazole Sulfonic Acid | 2,00 | | | | |
| TRIS AMINO ULTRA PURE | Thromethamine | 0,90 | | | | |
| NaOH 30% | Aqua, Sodium hydroxide | 0,90 | | | | |
| Citric acid 30% | Aqua, Citric acid | | | | | |
| TINOSORB®M | Methylene Bis-Benzotriazolyl Tetramethylbutylphenol | | 5,00 | 5,00 | 5,00 | 5,00 |
| TINOSORB®A2B | Tris-Biphenyl Triazine | | | | | |
| TINOSORB®S-AQUA | Bis-Ethylhexyloxyphenol Methoxyphenyl Triazine Polymethyl Methacrylate | | | | | |
| PHENONIP | Phenoxyethanol/Methylparaben/Ethylparaben/Butylparaben/Propylparaben | 1,00 | 1,00 | 1,00 | 1,00 | 1,00 |
| Aqua dem. | | Ad 100 | Ad 100 | Ad 100 | Ad 100 | Ad 100 |
| Aussehen (1 Tag) | | weiss | weiss | weiss | weiss | weiss |
| Struktur | | O/W Emulsion | O/W Emulsion | O/W Emulsion | O/W Emulsion | O/W Emulsion |
| SPF berechnet | | 59,9 | 60,2 | 60,2 | 60,2 | 60,2 |
| | | ratio 3,12 | ratio 2,59 | ratio 2,59 | ratio 2,59 | ratio 2,59 |

| Beispiel C24: SP15 | | | |
|---|---|---|---|
| Phase | Handelsname | INCI | Gew. % |
| **A** | Konzentrat nach Beispiel 6 | | 15.00 |
| B | Wasser | Aqua | 79.80 |
| | EDTA BD | Disodium EDTA | 0.20 |
| | TINOVIS ADE | Sodium Acrylates Copolymer/Hydrogenated Polydecene/PPG-1 Trideceth-6 | 3.20 |
| | NaOH 30% | Aqua, Sodium hydroxide | 0.80 |
| C | PHENONIP | Phenoxyethanol/Methylparaben/Ethylparab en/Butylparaben/Propylparaben/Isobutylparaben | 1.00 |
| SPF berechnet | | | 15.00 |

| Beispiel C25: SP 50+ | | | |
|---|---|---|---|
| Phase | Handelsname | INCI | Gew. % |
| A | CETIOL CC | Dicaprylyl Carbonate | 5.00 |
| | Konzentrat nach Beispiel 6 | | 50.00 |
| | NaOH 30% | Aqua, Sodium hydroxide | 2.50 |
| B | Wasser | Aqua | 29.80 |
| | EDTA BD | Disodium EDTA | 0.20 |
| | TINOVIS ADE | Sodium Acrylates Copolymer/Hydrogenated Polydecene/PPG-1 Trideceth-6 | 2.50 |
| C | DC246 | Cyclopentasiloxane | 2.00 |
| | DRY FLO PC | Aluminum Starch Octenylsuccinate | 2.00 |
| D | TINOSORB®M | Methylene Bis-Benzotriazolyl Tetramethylbutylphenol | 5.00 |
| E | PHENONIP | Phenoxyethanol/Methylparaben/Ethylparaben/Butylparaben/Propylparaben | 1.00 |
| | Parfum Jojoba oil | | |
| | SPF | | 57.50 |

| Beispiel C26: BB Cream | | | |
|---|---|---|---|
| Phase | Handelsname | INCI | Gew. % |
| A | Konzentrat nach Beispiel 6 | | 30.00 |
| B | Wasser | Aqua | |
| | Eumulgin BA25 | Beheneth-25 | 1.00 |
| | RHEOCARE XG | Xanthan Gum | 0.50 |
| | BUTYLENE GLYCOL | Butylene Glycol | 5.00 |
| C | DC 245 Fluid | Cyclopentasiloxane | 3.00 |
| D | Chione M-SVA | Synthetic Fluorphlogopite, Lauroyl Lysine | 1.00 |
| | Timica Terra White | Mica, Titanium Dioxide | 1.50 |
| | Timica Terra Yellow | Mica, Iron Oxides, Titanium Dioxide | 2.00 |
| | Timica Terra Red | Mica, Iron Oxides, Titanium Dioxide | 0.50 |
| | Timica Terra Black | Mica, Iron Oxides, Titanium Dioxide | 0.20 |
| | PHENONIP | PhenoxyethanolMethylparaben/Ethylparaben/Butylparaben/Propylparaben | 1.00 |
| | Parfum Cabano Rose | | 0.20 |
| | SPF | | 31.00 |

| Beispiel C27: Daily | | | |
|---|---|---|---|
| Phase | Handelsname | INCI | Gew .% |
| A | Konzentrat nach Beispiel 6 | | 15.00 |
| | Cetiol Sensoft | Propylheptyl Caprylate | 10.00 |
| B | Wasser | Aqua | 64.2 |
| | Glycerin 85% | Glycerin | 3.00 |
| | EDTA BD | Disodium EDTA | 0.20 |
| | Cosmedia SP | Sodium Polyacrylate | 0.40 |
| C | Tinovis ADE | Sodium Acrylates Copolymer/Hydrogenated Polydecene/PPG-1 Trideceth-6 | 1.50 |
| D | Orgasol Caresse | Polyamide-5 | |
| | Techpolymer MBP8 | Polymethyl Methacrylate | 2.00 |
| | DC245 | Cyclopentasiloxane | 2.00 |
| | Dermican SPB LS9837 | Glycerin, Aqua, Acetyl Tetrapeptide-9 | 0.50 |
| E | Parfum Cabano Rose | | 0.20 |
| F | PHENONIP | Phenoxyethanol/Methylparaben/Ethylparaben/Butylparaben/Propylparaben | 1.00 |
| | SPF | | 25.00 |

| Beispiel C28: BB Cream SPF30 | | | |
|---|---|---|---|
| Phase | Handelsname | INCI | Gew. % |
| A | Konzentrat nach Beispiel 6 | | 30.00 |
| | Eumulgin BA25 | Beheneth-25 | 1.00 |
| B | Wasser | Aqua | 40.10 |
| | RHEOCARE XG | Xanthan Gum | 0.50 |
| | BUTYLENE GLYCOL | Butylene Glycol | 5.00 |
| C | DC 245 Fluid | Cyclopentasiloxane | 0.50 |
| D | Chione M-SVA | Synthetic Fluorphlogopite (and) Lauroyl Lysine | 1.00 |
| | Timica Terra White | Mica, Titanium Dioxide | 3.00 |
| | Timica Terra Yellow | Mica, Iron Oxides, Titanium Dioxide | 4.00 |
| | Timica Terra Black | Mica, Iron Oxides, Titanium Dioxide | 0.40 |
| | PHENONIP | Phenoxyethanol/Methylparaben/Ethylparaben/Butylparaben/Propylparaben | 1.00 |

| Beispiel C29: Powder | | | |
|---|---|---|---|
| | Handelsname | INCI | Gew. % |
| | Konzentrat nach Beispiel 6 | | 5.00 |
| | Covi-ox T 90 EU | Tocopherol | 0.10 |
| | Cetiol C5 | Coco-Caprylate | 0.50 |
| | Cetiol B | Dibutyl Adipate | 0.70 |
| | Protectol PE | Phenoxyethanol | 0.50 |
| | Potassium sorbate | Potassium Sorbate | 0.30 |
| | Boroneige superfine | Boron Nitride | 5.00 |
| | Z cote HP1 | Zinc oxide (and) Triethoxycaprylylsilane | 20.00 |
| | KSP-411 | Polysilicone-22 | 5.00 |
| | Talc | Talc | 16.80 |
| | Chione M-SVA | Synthetic fluorphlogopite (and) Lauroyl lysine | 31.50 |
| | Timica Terra Yellow | Mica, Iron Oxides, Titanium Dioxide | 10.00 |
| | Timica Terra Red | Mica, Iron Oxides, Titanium Dioxide | 4.00 |
| | Timica Terra Black | Mica, Iron Oxides, Titanium Dioxide | 0.60 |

| Beispiel C30: WO/SPF 30 | | | |
|---|---|---|---|
| | Handelsname | INCI | Gew. % |
| A | Konzentrat nach Beispiel 6 | | 50.00 |
| | Dehymuls PGPH | Polyglyceryl-2 Dipolyhydroxystearate | 3.00 |
| | Lameform TGI | Polyglyceryl-3 Diisostearate | 1.00 |
| | Magnesium stearate | Magnesium Stearate | 1.00 |
| B | Wasser | Aqua | 35.00 |
| | Magnesium sulfate heptahydrate | Magnesium sulfate | 1.00 |
| | Glycerin 85% | Glycerin | 3.00 |
| C | Xiameter PMX-0345 | Cyclopentasiloxane (and) Cyclohexasiloxane | 5.00 |
| | Phenonip | Phenoxyethanol/Methylparaben/Ethylparaben/Butylparaben/Propylparaben | 1.00 |

| Beispiel C31: Lip Stick | | | |
|---|---|---|---|
| | Handelsname | INCI | Gew. % |
| A | Candelilla Wax | Candelilla Wax | 6.00 |
| | Carnauba Wax T1 | Carnauba Wax | 5.00 |
| | Performa V 1608 Polymer | C30-38 Olefin/Isopropyl Maleate/MA Copolymer | 4.00 |
| | Performa V 343 | Synthetic Wax | 4.00 |
| | Plandool-G | Bis-Behenyl/Isostearyl/ Phytosteryl Dimer Dilinoleyl Dimer Dilinoleate | 7.00 |
| B | Konzentrat nach Beispiel 6 | | 50.00 |
| | Nikkol DGTIS | Polyglyceryl-2 Triisostearate | 14.00 |
| | Cetiol SB 45 | Butyrospermum Parkii (Shea Butter) | 10.00 |
| C | Chione Digital pink | Synthetic Fluorphlogopite, Titanium Dioxide | 1.00 |
| | Parfum | | 0.50 |

## Patentansprüche

1. Hochkonzentrierte Mittel für die Herstellung von Sonnenschutz-Endformulierungen enthaltend
(a) 20 bis 35 Gew.% Ölkörper ausgewählt aus linearen und verzweigten Fettalkoholcarbonaten,
(b) 7 bis 17 Gew.% anionisches Tensid ausgewählt aus Alkylpolyalkylenglykolethercitraten,
(c) 3 bis 18 Gew.% weiteres, von (b) verschiedenes Co-Tensid ausgewählt aus Polyol- und/oder Polyglycerinestern;
(d) 35 bis 65 Gew.% mindestens eines öllöslichen UV-Filters; und
(e) 0,01 bis 5 Gew.-% Wasser sowie ggf. 0, 5 bis 5 Gew.-% Hilfsstoffe;
unter der Bedingung, dass sich alle Bestandteile auf 100 Gew.-% addieren.

2. Mittel nach Anspruch 1, **dadurch gekennzeichnet, dass** als Ölkörper (a) Dibutyladipate eingesetzt wird.

3. Mittel nach Anspruch 1 oder 2, **dadurch gekennzeichnet, dass** sie als anionisches Tensid (b) Alkylpolyalkylen Polyethylenglykolether des Laurylalkohols, enthalten.

4. Mittel nach Anspruch 1, **dadurch gekennzeichnet, dass** die öllöslichen UV-Filter (d) ausgewählt sind aus
(d_{SOL-1}) Benzophenone-3 (BP3);
(d_{SOL-2}) Benzophenone-4 (BP4);
(d_{SOL-3}) 3-Benzydilene Camphor (3BC);
(d_{SOL-4}) Bis-Ethylhexyloxyphenol Methoxyphenyl Triazine (BEMT);
(d_{SOL-5}) Butyl Methoxydibenzoylmethane (BMBM);
(d_{SOL-6}) Diethylhexyl Butamido Triazone (DBT);
(d_{SOL-7}) Drometrizole Trisiloxane (DTS);
(d_{SOL-8}) Ethylhexyl Triazone (EHT);
(d_{SOL-9}) Ethylhexyl Methoxycinnamate;
(d_{SOL-10}) Benzylidenemalonat (BM);
(d_{SOL-11}) Diethylamino Hydroxy Benzoyl Hexyl Benzoate (DHHB);
(d_{SOL-12}) Octocrylen;
(d_{SOL-13}) Polysilicon1-15;
(d_{SOL-14}) Homosalat; und
(d_{SOL-15}) Ethylhexylsalicylat.

5. Mittel nach Anspruch 1 oder 4, **dadurch gekennzeichnet dass** die öllöslichen UV-Filter (d) ausgewählt sind aus
(d₉ₐ) Ethylhexyl Methoxycinnamate,
(d₁₁ₐ) Bis-Ethylhexyloxyphenol Methoxyphenyl Triazine,
(d₁₃ₐ) Ethylhexyl Triazone und
(d₃ₐ) Diethylamino Hydroxy Benzoyl Hexyl Benzoate.

6. Mittel nach Anspruch 1, **dadurch gekennzeichnet, dass** sie
(a) 20 bis 35 Gew.-% eines Ölkörpers, ausgewählt aus linearen und verzweigten Fettalkoholcarbonaten, insbesondere Dicaprylyl Carbonate und Ester von C₂-C₁₂-Dicarbonsäuren mit linearen oder verzweigten Alkoholen;
(b) 7 bis 17 Gew.-% anionische Tenside, ausgewählt aus Alkylpolyalkylenglykolethercitraten, insbesondere die Polyethylenglykolether des Laurylalkohols;
(c) 3 bis 18 Gew-% Co-Tenside, ausgewählt aus nichtionischen Tensiden; vorzugsweise Polyol- und/oder Polyglycerinester;
(d) 35 bis 65 Gew.-% UV-Filter, ausgewählt aus
(d₉ₐ) Ethylhexyl Methoxycinnamate,
(d₁₁ₐ) Bis-Ethylhexyloxyphenol Methoxyphenyl Triazine,
(d₁₃ₐ) Ethylhexyl Triazone und
(d₃ₐ) Diethylamino Hydroxy Benzoyl Hexyl Benzoate; und
(e) 0,01 bis 5 Gew.-% Wasser sowie ggf. 0,5 bis 5 Gew.-% Hilfsstoffe enthalten;
wobei die Komponenten (d₉ₐ), (d₁₁ₐ), (d₁₃ₐ) und (d₃ₐ) als Einzelverbindung oder als 2-er, 3-er oder 4-er - Mischung in dem Mittel vorliegen können.

7. Verfahren zur Herstellung von Sonnenschutz-Endformulierungen enthaltend Mittel nach einem der Ansprüche 1 bis 6, **dadurch gekennzeichnet, dass** die Mittel mit Wasser, weiteren UV-Filtern sowie ggf. üblichen weiteren Hilfsstoffen bei Temperaturen im Bereich von 5°C bis 30° C verdünnt werden.

8. Verfahren nach Anspruch 7, **dadurch gekennzeichnet, dass** die hochkonzentrierten Mittel nach Anspruch 1 in Mengen von 6 bis 50 Gew.%, bezogen auf die Sonnenschutz-Endformulierung mit Wasser ggf. in Anwesenheit von Verdickern als Hilfsstoff verdünnt werden.

9. Verfahren nach Anspruch 8, **dadurch gekennzeichnet, dass** die Sonnennschutz-Endformulierungen als O/W-Emulsion, W/O-Emulsion, Gel, Gel-Creme, W/Si-Emulsion, Sprays, Cremes und Lotionen vorliegen.

10. Verwendung der Mittel nach einem der Ansprüche 1 bis 7 zur Herstellung von kosmetischen End-Formulierungen mit einem Sonnenschutzfaktor bis 50+ und mit UVA-PF/SPF > 1/3.

## Claims

1. A highly concentrated agent for the preparation of sunscreen final formulations, comprising
(a) 20 to 35 wt.% of oily substances selected from linear and branched fatty alcohol carbonates,
(b) 7 to 17 wt.% of anionic surfactant selected from alkyl polyalkylene glycol ether citrates,
(c) 3 to 18 wt.% of further co-surfactant which differs from (b) and is selected from polyol and/or polyglycerol esters;
(d) 35 to 65 wt.% of at least one oil-soluble UV filter; and
(e) 0.01 to 5 wt. % of water and also optionally 0.5 to 5 wt.% of auxiliary substances;
under the condition that all constituents add up to 100 wt.%.

2. The agent according to claim 1, **characterized in that** dibutyl adipate is employed as the oily substance (a).

3. The agent according to claim 1 or 2, **characterized in that** it comprises as the anionic surfactant (b) alkyl polyalkylene polyethylene glycol ether of lauryl alcohol.

4. The agent according to claim 1, **characterized in that** the oil-soluble UV filters (d) are selected from
(d_{SOL-1}) Benzophenone-3 (BP3);
(d_{SOL-2}) Benzophenone-4 (BP4);
(d_{SOL-3}) 3-Benzylidene Camphor (3BC);
(d_{SOL-4}) Bis-Ethylhexyloxyphenol Methoxyphenyl Triazine (BEMT);
(d_{SOL-5}) Butyl Methoxydibenzoylmethane (BMBM);
(d_{SOL-6}) Diethylhexyl Butamido Triazone (DBT);
(d_{SOL-7}) Drometrizole Trisiloxane (DTS);
(d_{SOL-8}) Ethylhexyl Triazone (EHT);
(d_{SOL-9}) Ethylhexyl Methoxycinnamate;
(d_{SOL-10}) Benzylidenemalonate (BM);
(d_{SOL-11}) Diethylamino Hydroxy Benzoyl Hexyl Benzoate (DHHB);
(d_{SOL-12}) Octocrylene;
(d_{SOL-13}) Polysiliconel-15;
(d_{SOL-14}) Homosalate; and
(d_{SOL-15}) Ethylhexyl salicylate.

5. The agent according to claim 1 or 4, **characterized in that** the oil-soluble UV filters (d) are selected from
(d₉ₐ) Ethylhexyl Methoxycinnamate,
(d₁₁ₐ) Bis-Ethylhexyloxyphenol Methoxyphenyl Triazine,
(d₁₃ₐ) Ethylhexyl Triazone and
(d₃ₐ) Diethylamino Hydroxy Benzoyl Hexyl Benzoate.

6. The agent according to claim 1, **characterized in that** it comprises
(a) 20 to 35 wt.% of an oily substance selected from linear and branched fatty alcohol carbonates, in particular Dicaprylyl Carbonate and esters of C₂-C₁₂-dicarboxylic acids with linear or branched alcohols;
(b) 7 to 17 wt.% of anionic surfactants selected from alkyl polyalkylene glycol ether citrates, in particular the polyethylene glycol ether of lauryl alcohol;
(c) 3 to 18 wt.% of co-surfactants selected from nonionic surfactants; preferably polyol and/or polyglycerol esters;
(d) 35 to 65 wt.% of UV filters selected from
(d₉ₐ) Ethylhexyl Methoxycinnamate,
(d₁₁ₐ) Bis-Ethylhexyloxyphenol Methoxyphenyl Triazine,
(d₁₃ₐ) Ethylhexyl Triazone and
(d₃ₐ) Diethylamino Hydroxy Benzoyl Hexyl Benzoate; and
(e) 0.01 to 5 wt.% of water and optionally 0.5 to 5 wt.% of auxiliary substances;
wherein components (d₉ₐ), (d₁₁ₐ), (d₁₃ₐ) and (d₃ₐ) can be present in the agent as an individual compound or a 2-, 3- or 4-component mixture.

7. A process for the preparation of sunscreen final formulations comprising agents according to one of claims 1 to 6, **characterized in that** the agents are diluted with water, optionally further UV filters and optionally conventional further auxiliary substances at temperatures in the range of from 5 to 30 °C.

8. The process according to claim 7, **characterized in that** the highly concentrated agents as claimed in claim 1 are diluted in amounts of from 6 to 50 wt.%, based on the sunscreen final formulation, with water, optionally in the presence of thickeners as an auxiliary substance.

9. The process according to claim 8, **characterized in that** the sunscreen final formulations are in the form of an O/W emulsion, W/O emulsion, gel, gel-cream, W/Si emulsion, sprays, creams and lotions.

10. The use of the agents according to one of claims 1 to 7 for the preparation of cosmetic final formulations having a sun protection factor of up to 50+ and a UVA-PF/SPF of > 1/3.

## Revendications

1. Agents hautement concentrés pour la fabrication de formulations finales de protection solaire, contenant :
(a) 20 à 35 % en poids de corps huileux choisis parmi les carbonates d'alcools gras linéaires et ramifiés,
(b) 7 à 17 % en poids d'un tensioactif anionique choisi parmi les éther-citrates d'alkylpolyalkylène glycol,
(c) 3 à 18 % en poids d'un co-tensioactif supplémentaire, différent de (b), choisi parmi les esters de polyol et/ou de polyglycérine ;
(d) 35 à 65 % en poids d'au moins un filtre UV soluble dans les huiles ; et
(e) 0,01 à 5 % en poids d'eau et éventuellement 0,5 à 5 % en poids d'adjuvants ;
à condition que la somme de tous les constituants soit de 100 % en poids.

2. Agents selon la revendication 1, **caractérisés en ce que** de l'adipate de dibutyle est utilisé en tant que corps huileux (a).

3. Agents selon la revendication 1 ou 2, **caractérisés en ce qu'**ils contiennent en tant que tensioactif anionique (b) des éthers d'alkylpolyalkylène polyéthylène glycol de l'alcool laurylique.

4. Agents selon la revendication 1, **caractérisés en ce que** les filtres UV solubles dans les huiles (d) sont choisis parmi :
(d_{SOL-1}) Benzophenone-3 (BP3) ;
(d_{SOL-2}) Benzophenone-4 (BP4) ;
(d_{SOL-3}) 3-Benzylidene Camphor (3BC) ;
(d_{SOL-4}) Bis-Ethylhexyloxyphenol Methoxyphenyl Triazine (BEMT) ;
(d_{SOL-5}) Butyl Methoxydibenzoylmethane (BMBM) ;
(d_{SOL-6}) Diethylhexyl Butamido Triazone (DBT) ;
(d_{SOL-7}) Drometrizole Trisiloxane (DTS) ;
(d_{SOL-8}) Ethylhexyl Triazone (EHT) ;
(d_{SOL-9}) Ethylhexyl Methoxycinnamate ;
(d_{SOL-10}) Benzylidenemalonate (BM) ;
(d_{SOL-11}) Diethylamino Hydroxy Benzoyl Hexyl Benzoate (DHHB) ;
(d_{SOL-12}) Octocrylene ;
(d_{SOL-13}) Polysilicone 1-15 ;
(d_{SOL-14}) Homosalate ; et
(d_{SOL-15}) Ethylhexyl salicylate.

5. Agents selon la revendication 1 ou 4, **caractérisés en ce que** les filtres UV solubles dans les huiles (d) sont choisis parmi :
(d₉ₐ) Ethylhexyl Methoxycinnamate,
(d₁₁ₐ) Bis-Ethylhexyloxyphenol Methoxyphenyl Triazine,
(d₁₃ₐ) Ethylhexyl Triazone et
(d₃ₐ) Diethylamino Hydroxy Benzoyl Hexyl Benzoate.

6. Agents selon la revendication 1, **caractérisés en ce qu'**ils contiennent :
(a) 20 à 35 % en poids d'un corps huileux, choisi parmi les carbonates d'alcools gras linéaires et ramifiés, notamment le Dicaprylyl Carbonate et les esters d'acides dicarboxyliques en C₂-C₁₂ avec des alcools linéaires ou ramifiés ;
(b) 7 à 17 % en poids de tensioactifs anioniques choisis parmi les éther-citrates d'alkylpolyalkylène glycol, notamment l'éther de polyéthylène glycol de l'alcool laurylique,
(c) 3 à 18 % en poids de co-tensioactifs, choisis parmi les tensioactifs non ioniques, de préférence les esters de polyol et/ou de polyglycérine ;
(d) 35 à 65 % en poids de filtres UV, choisis parmi :
(d₉ₐ) Ethylhexyl Methoxycinnamate,
(d₁₁ₐ) Bis-Ethylhexyloxyphenol Methoxyphenyl Triazine,
(d₁₃ₐ) Ethylhexyl Triazone et
(d₃ₐ) Diethylamino Hydroxy Benzoyl Hexyl Benzoate ; et
(e) 0,01 à 5 % en poids d'eau et éventuellement 0,5 à 5 % en poids d'adjuvants ;
les composants (d₉ₐ), (d₁₁ₐ), (d₁₃ₐ) et (d₃ₐ) pouvant être présents dans l'agent sous la forme d'un composé individuel ou d'un mélange de 2, 3 ou 4.

7. Procédé de fabrication de formulations finales de protection solaire contenant des agents selon l'une quelconque des revendications 1 à 6, **caractérisé en ce que** les agents sont dilués avec de l'eau, des filtres UV supplémentaires, ainsi qu'éventuellement des adjuvants usuels supplémentaires à des températures dans la plage allant de 5 °C à 30 °C.

8. Procédé selon la revendication 7, **caractérisé en ce que** les agents hautement concentrés selon la revendication 1 sont dilués en quantités de 6 à 50 % en poids, par rapport à la formulation finale de protection solaire, avec de l'eau, éventuellement en présence d'épaississants en tant qu'adjuvant.

9. Procédé selon la revendication 8, **caractérisé en ce que** les formulations finales de protection solaire se présentent sous la forme d'une émulsion H/E, d'une émulsion E/H, d'un gel, d'un gel-crème, d'une émulsion E/Si, d'une pulvérisation, d'une crème et d'une lotion.

10. Utilisation des agents selon l'une quelconque des revendications 1 à 7 pour la fabrication de formulations finales cosmétiques ayant un facteur de protection solaire de jusqu'à 50+ et un UVA-PF/SPF > 1/3.
